# EUROPÄISCHE PATENTSCHRIFT

(11) **EP 1 479 355 B1**
(45) Veröffentlichungstag und Bekanntmachung des Hinweises auf die Patenterteilung: **05.07.2017**
(21) Anmeldenummer: 04011999.2
(22) Anmeldetag: 21.05.2004
(51) Int. Cl.: A41D 19/00

(54) **Prophylaxeartikel**
Device for prophylaxis
Dispositif de prophylaxie

(30) Priorität: 21.05.2003 AT 7862003
(43) Veröffentlichungstag der Anmeldung: 24.11.2004
(73) Patentinhaber: Semperit Aktiengesellschaft Holding, 1031 Wien (AT)
(72) Erfinder: Schaller, Raimund, Dipl.-Ing. Dr., 2620 Neunkirchen (AT)
(74) Vertreter: Ofner, Clemens

(56) Entgegenhaltungen:
- EP-A- 0 443 870
- EP-A- 0 824 896
- WO-A-99/19006
- US-A- 4 143 109
- US-A- 4 930 522
- US-A- 5 138 719
- US-A1- 2003 021 903

## Beschreibung

Die Erfindung betrifft einen mehrschichtigen Prophylaxeartikel, insbesondere einen medizinischen Handschuh, aus einer elastomeren Trägerschicht, wie z.B. einem Synthese- oder Naturlatex, mit einer inneren und einer äußeren Oberfläche, wobei auf der inneren Oberfläche zumindest in einem Teilbereich eine Gleitschicht aus einem polymeren Material mit einer inneren und einer, der inneren Oberfläche der Trägerschicht zugewandten, äußeren Oberfläche angeordnet ist, sowie ein Verfahren zu dessen Herstellung.

Medizinische Handschuhe, sei es in Form von Untersuchungshandschuhen oder in Form von Operationshandschuhen gehören mittlerweile zum standardmäßigen Equipment der medizinischen Versorgung. Problematisch hierbei ist, dass, wenn derartige Handschuhe über einen längeren Zeitraum getragen werden, die Haut des Verwenders unter Umständen mit Irritationen oder allergisch auf die diversen Elastomermaterialien reagiert. Um dies zu vermeiden, wurde im Stand der Technik bereits vorgeschlagen, diverse Substanzen über den Handschuh auf die Haut aufzubringen.

So ist z.B. aus der US 6,274,154 B1 ein therapeutischer Handschuh, der aus einer einzigen Schicht aus einem flexiblen Material gebildet ist, und der auf seiner inneren Oberfläche eine Schicht aus dehydratisierter Aloe Vera aufweist, bekannt. Die Aloe Vera Schicht wird über einen Tauchprozess aus einer diese umfassenden Lösung aufgetaucht und der Handschuh wird in der Folge erhitzt, um die Dehydratisierung und die Ausbildung der Aloe Vera Schicht zu erreichen.

Andererseits ist es aus dem Stand der Technik bekannt, Wirkstoffe mikroverkapselt in Polyacrylat - Polyurethan- Copolymer Dispersionen vorzulegen und über einen Tauchprozess aus diesen Dispersionen einen Handschuh zu fertigen. Beispielsweise beschreibt die DE 201 00 269 U1 einen beschichteten, medizinischen Handschuh mit synergistischer Anti-HIV-Wirkung, wobei antivirale Substanzen durch Mikrokapseltechnik in kugelförmigen Einzelkapseln als Füllstoff enthalten sind. Die Aktivierung der Substanzwirkstoffe erfolgt unmittelbar nach Zerbersten der Kapseln unter Druckeinfluss.

Es ist weiters aus der US 4,775,372 A ein Aloe Vera Öl enthaltender Handschuh bekannt, wobei das Öl zwischen zwei Schichten aus flexiblen Kunststoff, welche Taschen durch Beabstandung der beiden Kunststoffschichten zueinander ausbilden, angeordnet ist. Zur Applikation des Öls müssen diese Taschen zumindest punktiert werden, sodass der Handschuh nicht nur seine Festigkeit zumindest bereichsweise verliert, sondern auch damit die Gefahr verbunden ist, dass Substanzen von Außen in die Haut des Verwenders eindringen.

Es ist Aufgabe der Erfindung einen Prophylaxeartikel mit verbesserten Eigenschaften zu schaffen. Insbesondere soll der Prophylaxeartikel zum einen eine verbesserte Anziehbarkeit, aufweisen, und zum anderen Wirkstoffe, gegebenenfalls bereits während des Anziehens des Artikels, freisetzen.

Die Aufgabe der Erfindung wird jeweils eigenständig durch einen eingangs erwähnten mehrschichtigen Prophylaxeartikel gelöst, bei dem auf der inneren Oberfläche der Trägerschicht und/oder zwischen der Trägerschicht und der Gleitschicht und/oder in der Gleitschicht und/ oder auf der inneren Oberfläche der Gleitschicht zumindest im Teilbereich zumindest ein Wirkstoff und/oder Farbstoff in Partikeln, insbesondere Mikrokapseln, mit einem maximalen Durchmesser ausgewählt aus einem Bereich mit einer oberen Grenzen von 500 µm, insbesondere 400 µm, vorzugsweise 300 µm und einer unteren Grenze von 10 µm, vorzugsweise 30 µm, insbesondere 40 µm, angeordnet ist, und/oder bei dem in dem zumindest einen Teilbereich zwischen der Trägerschicht und der Gleitschicht eine Schicht umfassend den zumindest einen Wirkstoff und/oder Farbstoff angeordnet ist, wobei die Gleitschicht regelmäßig wiederkehrende Erhöhungen bzw. Vertiefungen mit unregelmäßiger Form, hergestellt durch raschen Flüssigkeitsentzug aus der Gleitschicht, aufweist, wobei sich ein Anteil der Vertiefungen, ausgewählt aus einem Bereich mit einer unteren Grenze von 20 %, insbesondere 35 %, vorzugsweise 40 %, und einer oberen Grenze von 95 %, insbesondere 80 %, vorzugsweise 75 %, bezogen auf die Gesamtzahl der Vertiefungen, über die gesamte Dicke der Gleitschicht erstreckt, sowie durch ein eingangs erwähntes Verfahren, welches die Herstellung des erfindungsgemäßen Prophylaxeartikels, ermöglicht, nach dem der Wirkstoff und/oder Farbstoff insbesondere durch Tauchen oder Aufsprühen aufgebracht wird. Von Vorteil erweist sich dabei, dass durch die erzielte Oberflächenrauhigkeit der Gleitschicht durch die Partikel bzw. Erhöhungen bzw. Vertiefungen nicht nur eine bessere Anziehbarkeit des Handschuhs an sich ermöglicht wird, sondern gleichzeitig auch eine verbesserte Nassschlüpfrigkeit erzielt werden kann. Dadurch wird der Vorteil erreicht, dass im Vergleich mit herkömmlichen Handschuhen ohne diese Nassschlüpfrigkeit die Wirkstoffe und/oder Farbstoffe über einen längeren Zeitraum an die Haut abgegeben werden können und nicht sofort beim Anziehen des Handschuhs aufgrund der mechanischen Belastung, insbesondere Reibung, zur Gänze freigesetzt werden. Weiters erweist es sich von Vorteil, dass die Partikel den Effekt der besseren Anziehbarkeit eines Prophylaxeartikels, der bereits durch sein Herstellungsverfahren eine gewisse Oberflächenrauhigkeit aufweisen kann, noch verstärken. Des weiteren erweist sich von Vorteil, dass durch die Oberflächenrauhigkeit kein vollflächiger Kontakt zwischen dem Prophylaxeartikel und der menschlichen Haut besteht und es dadurch zu einer verminderten Schweißbildung kommt bzw. der gebildete Schweiß leichter abtransportiert werden kann und somit der Tragekomfort für den Träger des Prophylaxeartikels erhöht wird. Durch die kleinere Berührungsfläche zwischen dem Prophylaxeartikel und der menschlichen Haut wird weiters die Verträglichkeit des Prophylaxeartikels gesteigert und die Häufigkeit des Auftretens von allergischen Reaktionen vermindert bzw. bei geeigneter Auswahl der Wirkstoffe zumindest annähernd zur Gänze vermieden. Von Vorteil erweist sich des weiteren auch, dass beim An- bzw. Überziehen des Prophylaxeartikels es zu einer Dehnung kommt und in der Folge dessen Wandstärke derart verringert wird, dass die Oberflächenrauhigkeit aufgrund der weiter hervortretenden Partikel verstärkt wird und das Anziehen zusätzlich erleichtert wird.

Gemäß einer Weiterbildung der genannten Erfindung, wonach der Durchmesser der Partikel aus einem Bereich mit einer oberen Grenze von 250 µm, vorzugsweise 200 µm, insbesondere 150 µm und einer unteren Grenze von 50 µm, vorzugsweise 80 µm, insbesondere 100 µm, ausgewählt ist, bleibt das Tastgefühl, insbesondere wenn die Teilbereiche die Endglieder der Finger mit umfassen, vollständig erhalten.

Vorteilhaft ist weiters, wenn der Durchmesser der Partikel zumindest 80 % der Dicke der Gleitschicht umfasst, weil dadurch das Herstellungsverfahren für erfindungsgemäße Prophylaxeartikel insofern vereinfacht werden kann, als nicht auf eine homogene Verteilung der Partikel in einem Gemisch, mit welchem die Partikel aufgetragen werden können, geachtet werden muss und trotzdem die Oberflächenrauhigkeit der inneren Oberfläche des Prophylaxeartikels gewährleistet und damit die Nassschlüpfrigkeit verbessert werden kann.

Dieser eben genannte Effekt wird dadurch verstärkt, wenn die Partikel den gleichen Durchmesser wie die Dicke der Gleitschicht aufweisen

Die Partikel können einen größeren Durchmesser als die Dicke der Gleitschicht aufweisen, wodurch ein größeres Volumen an Wirkstoffen und/oder Farbstoffen in den Partikeln enthalten sein kann.

Des weiteren erweist sich als vorteilhaft, dass der Teilbereich des Prophylaxeartikels den Bereich des distalen Unterarms, der Handwurzelknochen, der Mittelhandknochen, der Grund-, Mittel- und Endglieder der Finger, umfasst, wobei die Wirkstoffe und/oder Farbstoffe ganz gezielt an die jeweiligen Körperteile, wie z.B. Flächen der Hand, abgegeben werden können und dadurch die Haut im jeweiligen Bereich ausreichend mit den Wirkstoffen versorgt wird.

Gemäß einer Weiterbildung ist vorgesehen, dass die Partikel sowohl palmar als auch dorsal in zumindest einem Teilbereich des Prophylaxeartikels aufgebracht sind, wodurch nicht nur der erhöhte Feuchtigkeitsbedarf der dorsalen Handseite gedeckt werden kann, sondern auch die vermehrte Transpiration an der palmaren Seite vermieden werden kann.

Von Vorteil ist auch, wenn sich der Teilbereich über einen Bereich der inneren Oberfläche der Trägerschicht und/oder zwischen der Trägerschicht und der Gleitschicht und/oder in der Gleitschicht und/oder auf der inneren Oberfläche der Gleitschicht mit einer unteren Grenze von 40 %, vorzugsweise 50 %, insbesondere 60 %, und einer oberen Grenze von 90 %, vorzugsweise 80 %, insbesondere 70 %, erstreckt, wodurch durch die großflächige Aufbringung der Wirkstoffe und/oder Farbstoffe die Sicherheit für den Träger des Prophylaxeartikels in Hinblich auf die Wirkstoffabgabe erhöht werden kann.

Die Partikel können eine andere Farbe als die Träger- bzw. die Gleitschicht aufweisen, wodurch die Verteilung der Partikel im Prophylaxeartikel überprüft werden kann. Von Vorteil erweist sich dabei weiters, dass das Aufplatzen der Partikel unter Druckbelastung visuell beobachtet werden kann und dadurch für den Verwender des Prophylaxeartikels eine Gewährleistung der Freisetzung der Wirkstoffe und/oder Farbstoffe gegeben ist.

Weiters erweist sich von Vorteil, dass die Partikel wasserunlöslich sind, wodurch diese über ein Tauchverfahren aufgebracht werden können und die Wirkstoffe bzw. die Farbstoffe nicht bereits während der Herstellung des Prophylaxeartikels in den Tauchschritten bzw. anschließenden Waschschritten freigesetzt werden.

Gemäß einer weiteren Ausführungsvariante ist vorgesehen, dass die Partikel wasserlöslich sind, wodurch diese auch in einem Sprühverfahren aufgebracht werden können und somit ein weiteres Herstellungsverfahren zur Herstellung des Prophylaxeartikels zur Verfügung steht. Weiters erweist sich dabei von Vorteil, dass bereits durch den Kontakt der Partikel mit flüssigem Schweiß des Verwenders eine Freisetzung der Wirkstoffe und/oder Farbstoffe erfolgen kann und keine Druckbelastung zum Freisetzen der Wirkstoffe und/oder Farbstoffe notwendig ist, sodass eine Wirkstoffabgabe auch dann noch möglich ist, wenn der Verwender eine unpassende Größe des Prophylaxeartikels wählt.

Die Wirkstoffe können antibakterielle bzw. antivirale bzw. antitranspirante bzw. spermizide bzw. pflegende Wirkung aufweisen, sodass durch die Verwendung des Prophylaxeartikels sehr viele verschiedene Wirkungen erzielt werden können. Dabei erweist sich von Vorteil, dass durch die antibakterielle bzw. antivirale Wirkung Infektionen viraler und bakterieller Natur bei Zerstörungen bzw. Verletzungen des Prophylaxeartikels dennoch vermieden werden können. Durch die antitranspirante Wirkung der Wirkstoffe wird eine verminderte Schweißbildung des Verwenders bewirkt und dadurch der Tragekomfort des Prophylaxeartikels vergrößert. Durch die spermizide Wirkung der Wirkstoffe kann beispielsweise bei Verletzungen bzw. Zerplatzen eines Kondoms die kontrazeptive Wirkung dennoch aufrecht erhalten werden.

Die Wirkstoffe können aus einer Gruppe umfassend Chlorhexidin, z.B. ein Gluconat, ein Acetat, ein Hydrochlorid, Nonoxinol 9 und Aloe Vera ausgewählt sein, womit dem Prophylaxeartikel eine antiseptische und desinfizierende Wirkung gegen eine Vielzahl von unterschiedlichen Bakterien und Pilzen, sowie einigen Viren, verliehen werden kann. Zudem sind diese Wirkstoffe weitestgehend unbedenklich und weisen teilweise darüber hinaus, hautpflegende Eigenschaften auf.

Die Wirkstoffe können weiters aus einer Gruppe umfassend Vitamine, Pflanzeninhaltsstoffe, insbesondere sekundäre Pflanzeninhaltsstoffe, ausgewählt sein, wonach insbesondere pflegende Eigenschaften des Prophylaxeartikels induziert werden.

In einer Weiterbildung der eben genannten Ausführungsvariante können die Vitamine aus einer Gruppe umfassend Verbindungen mit Retinoidstruktur (Vitamine A), Vitamin-B-Komplex, Ascorbinsäure (Vitamine C), Calciferole (Vitamine D), Tocopherole (Vitamine E), Vitamine K, Flavanoide und Biotin ausgewählt sein, wonach eine sehr individuelle Versorgung mit Wirkstoffen dem jeweiligen Verwender je nach Bedarf zur Verfügung gestellt werden kann.

Die Konzentration des zumindest einen Wirkstoffs und/oder Farbstoffs in einem Partikel kann aus einem Bereich mit einer unteren Grenze von 1 %, vorzugsweise 2 %, insbesondere 5 % und einer oberen Grenze von 10 %, vorzugsweise 15 %, insbesondere 20 % ausgewählt sein, wodurch einerseits kein übermäßiges Maß an Wirkstoff und/oder Farbstoff auf die Haut des Verwenders des Prophylaxeartikels aufgebracht wird und dadurch keine Überversorgung zustande kommt und andererseits eine kostenbewusste Herstellung des Prophylaxeartikels ermöglicht wird.

Dadurch, dass die Hülle der Partikel gemäß einer Weiterbildung druckempfindlich ist, wird durch Druckbelastung der zumindest eine Wirkstoff und/oder Farbstoff freigesetzt, wodurch bereits ein Teil des Wirkstoffes und/oder Farbstoffes beim Anziehen des Prophylaxeartikels freigegeben werden kann und in weiterer Folge zumindest weitestgehend homogen über die gesamte Hand bzw. den Körperteil, über welchen der Prophylaxeartikel gezogen wird, durch das Überstreifen des Prophylaxeartikels verteilt wird.

Von Vorteil ist, wenn die Partikel in dem zumindest einen Teilbereich die Gleitschicht bilden, wodurch der Effekt der Oberflächenrauhigkeit und damit die Anziehbarkeit des Prophylaxeartikels weiters verbessert werden kann.

Die Dicke der Gleitschicht kann aus einem Bereich mit einer unteren Grenze von 30 µm, vorzugsweise 40 µm, insbesondere 50 µm, und einer oberen Grenze von 500 µm, vorzugsweise 400 µm, insbesondere 300 µm, ausgewählt sein, wobei in einer Ausführungsvariante hierzu sich der Bereich an einer unteren Grenze von 55 µm, vorzugsweise 60 µm, insbesondere 75 µm und einer oberen Grenze von 200 µm, vorzugsweise 150 µm, insbesondere 110 µm, erstrecken kann, wodurch sich eine weitere Optimierung der Anziehbarkeit insbesondere der Nassschlüpfrigkeit des Prophylaxeartikels erreichen lässt.

Gemäß einer Ausführungsvariante ist vorgesehen, dass die Vertiefungen einen maximalen Durchmesser, in Draufsicht gesehen, ausgewählt aus einem Bereich mit einer oberen Grenze von 30 µm, vorzugsweise 25 µm, insbesondere 20 µm, und einer unteren Grenze von 1 µm, vorzugsweise 5 µm, insbesondere 10 µm, aufweisen. Diese Vertiefungen können dabei nach einer Weiterbildung kraterförmig sich in Richtung auf die Trägerschicht verjüngend ausgebildet sein, wobei in einer bevorzugten Ausführungsvariante die Wände der kraterförmigen Vertiefungen einen Neigungswinkel, bezogen auf die Normale auf die Gleitschicht aufweisen können, der aus einem Bereich mit einer unteren Grenze von 30°, insbesondere 42°, vorzugsweise 47°, und einer oberen Grenze von 80°, insbesondere 75°, vorzugsweise 60°, ausgewählt sein kann. Durch eine derartige Ausgestaltung der Vertiefungen ist eine verbesserte Abgabe des Wirkstoffes und/oder Farbstoffes erreichbar und ist zum Anderen auch eine verbesserte Schweißabfuhr während des Tragens von der Haut in den Prophylaxeartikel erzielbar, sodass der Tragekomfort weiter verbessert werden kann, insbesondere auch die Ausziehbarkeit des Prophylaxeartikels, da die Haftung desselben an der Haut in geringerem Ausmaß stattfindet.

Von Vorteil ist es, wenn die Menge des Wirkstoffes und/oder des Farbstoffes so bemessen ist, dass der Wirkstoff und/oder Farbstoff über die gesamte Tragedauer des Prophylaxeartikels in vorzugsweise zumindest annähernd gleichmäßigen Dosen abgegeben wird, da damit die Funktionalität des Prophylaxeartikels über die gesamte Tragedauer gewährleistet und auch eine zeitweise Überdosierung des Wirkstoffes und/oder Farbstoffes verhindert werden kann.

Die Löslichkeit bei 20°C die der Wirkstoff und/oder Farbstoff in Wasser aufweisen kann, ist vorzugsweise aus einem Bereich mit einer unteren Grenze von 1 g/l, vorzugsweise 3 g/l, insbesondere 4 g/l, und einer oberen Grenze von 20 g/l, vorzugsweise 15 g/l, insbesondere 8 g/l, ausgewählt ist, sodass die Sicherheit, mit der der Wirkstoff und/oder Farbstoff abgegeben wird und über die entsprechende Flüssigkeit, insbesondere den Schweiß, der Haut des Verwenders des Prophylaxeartikels zugeführt wird, weiter verbessert werden kann.

Es ist weiters möglich, dass eine Lösung des Wirkstoffes und/oder Farbstoffes im Partikel einen pH-Wert ausgewählt aus einem Bereich von 5,5 bis 7,5 aufweist, sodass eine gute Hautverträglichkeit des Prophylaxeartikels erhalten wird.

Nach Ausführungsvarianten ist vorgesehen, dass die Erhebungen, zumindest großteils netzförmig mit miteinander verbundenen Stegen ausgebildet sind, wobei eine Höhe zumindest eines Teils der Stege einen Wert aufweist, der im Bereich zwischen 25 % und 100 %, vorzugsweise 33 % und 75 %, insbesondere 40 % und 60 %, der Gesamtdicke der Gleitschicht beträgt. Es ist damit eine entsprechende Reduzierung des direkten Kontaktes und damit der Haftung des Prophylaxeartikels auf der Haut erreichbar und steht damit weiters eine ausreichende Anzahl an "Poren", über welche der Wirkstoff und/oder Farbstoff aus dem inneren des Prophylaxeartikels an die Haut herangeführt wird, zur Verfügung.

Die Partikel können in Form eines heterogenen Gemisches, insbesondere einer Suspension bzw. Dispersion, aufgebracht werden, wodurch die Partikel nach einem standardisierten Verfahren verarbeitet werden können.

Zumindest ein Teil des heterogenen Gemisches, insbesondere die Partikel, kann in dem zumindest einen Teilbereich die Gleitschicht ausbilden, wonach die Wirkstoffe und/oder Farbstoffe direkt mit der Hautoberfläche des Verwenders des Prophylaxeartikels in Kontakt treten und vorher nicht erst die Gleitschicht durchdringen müssen.

Gemäß Weiterbildungen der Erfindung wird die Konzentration der Partikel des heterogenen Gemischs aus einem Bereich mit einer unteren Grenze von 1 %, insbesondere 2 %, vorzugsweise 5 % und einer oberen Grenze von 50 %, vorzugsweise 40 %, insbesondere 30 % ausgewählt bzw. aus einem Bereich mit einer unteren Grenze von 6 %, vorzugsweise 7 %, insbesondere 10 % und einer oberen Grenze von 25 %, vorzugsweise 20 %, insbesondere 15 % ausgewählt, wodurch eine ausreichende Menge an Wirkstoffen dem Verwender des Prophylaxeartikels zur Verfügung gestellt wird. Weiters erweist sich dabei von Vorteil, dass die Konzentration so ausgewählt wird, dass eine kostenbewusste Herstellung des Prophylaxeartikels ermöglicht wird.

Das erfindungsgemäße Verfahren kann derart gesteuert sein, dass der Flüssigkeitsentzug innerhalb einer Zeitspanne mit einer unteren Grenze von 10 sec., insbesondere 25 sec., vorzugsweise 50 sec., und einer oberen Grenze von 20 min., insbesondere 15 min., vorzugsweise 10 min., erfolgt. Es erweist sich auch von Vorteil, wenn der Flüssigkeitsentzug bei einer Temperatur, ausgewählt aus einem Bereich mit einer unteren Grenze von 60°C, insbesondere 66°C, vorzugsweise 70°C, und einer oberen Grenze von 150°C, insbesondere 125°C, vorzugsweise 110°C, durchgeführt wird. Durch diese Weiterbildungen kann eine Verbesserung im Hinblick auf die entstehenden Vertiefungen bzw. Erhebungen erreicht werden, sodass die Trage-, Anzieh- und Auszieheigenschaften des nach dem Verfahren hergestellten Prophylaxeartikels verbessert sind.

Da die weiteren Ausführungsvarianten des Verfahrens zur Herstellung der entsprechenden Ausführungsvarianten des Prophylaxeartikels dienen und die Merkmale dieser Ansprüche übertragen dem Sinn der jeweiligen beanspruchten Weiterbildungen des Prophylaxeartikels entsprechen, soll, zur Vermeidung von unnötigen Wiederholungen, an dieser Stelle auf die jeweiligen voranstehenden Ausführungen verwiesen sein, wobei die mit den Weiterbildungen erzielten Vorteile entsprechend übertragen werden können.

Zum besseren Verständnis wird die Erfindung anhand der nachfolgenden Figuren näher erläutert.

Es zeigen jeweils in schematisch vereinfachter Darstellung:
- Fig. 1: einen Prophylaxeartikel in Form eines Handschuhs;
- Fig. 2: den Querschnitt des Prophylaxeartikels gemäß der Schnittlinie II-II in Fig.1;
- Fig. 3: einen Teil des Prophylaxeartikels geschnitten entsprechend den Schnittlinien III-III in Fig. 2;
- Fig. 4: ein Detail einer Ausführungsvariante des Prophylaxeartikels gemäß Fig. 3 in Schnittdarstellung;
- Fig. 5: ein Detail einer weiteren Ausführungsvariante des Prophylaxeartikels gemäß Fig. 3 in Schnittdarstellung;
- Fig. 6: ein Detail einer weiteren Ausführungsvariante des Prophylaxeartikels gemäß Fig. 3 in Schnittdarstellung;
- Fig. 7: eine Explosionsdarstellung eines Ausschnittes einer Ausführungsvariante des Prophylaxeartikels, insbesondere eine Detailansicht.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind bei einer Lageänderung sinngemäß auf die neue Lage zu übertragen. Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

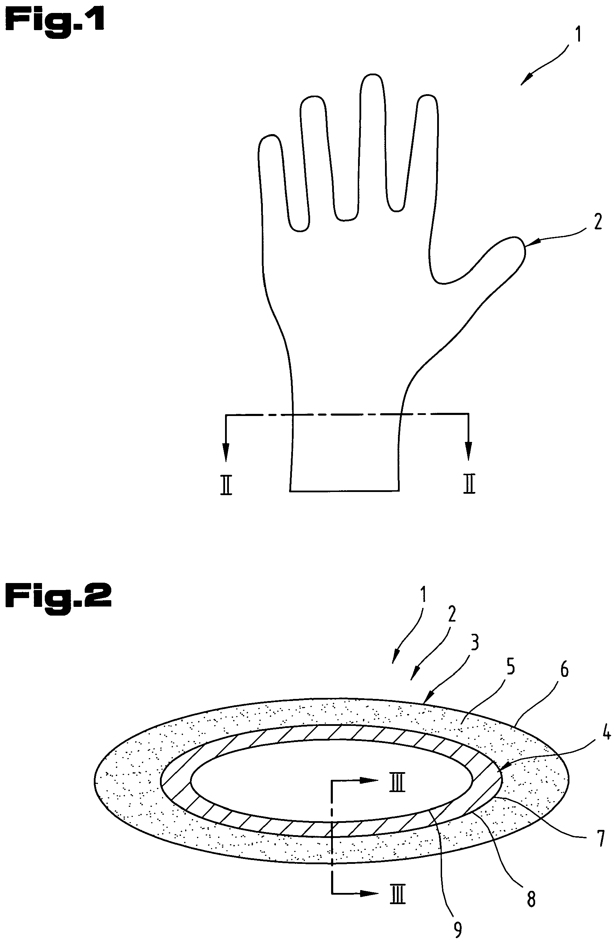

In den Fig. 1 bis 3 ist ein erfindungsgemäßer Prophylaxeartikel 1, in Form eines Handschuhs 2 dargestellt.

Bei dem Prophylaxeartikel 1 kann es sich neben Handschuhen 2, insbesondere medizinische Operations- und Untersuchungshandschuhe, auch um Katheder, Kondome, Fingerlinge, Badehauben, Schwimmflossen oder Schutzhandschuhe für die Arbeit in Reinraumbereichen etc. handeln.

Fig. 2 zeigt im Querschnitt gemäß der Schnittlinie II-II aus Fig.1 den Aufbau einer Ausführungsvariante des Prophylaxeartikels 1.

Der Prophylaxeartikel 1 besteht aus einem Trägermaterial 3 und einer darauf angeordneten Gleitschicht 4. Das Trägermaterial 3 bildet zumindest eine Trägerschicht 5. Selbstverständlich ist es möglich dass mehrere Trägerschichten 5 angeordnet werden, wobei diese Trägerschichten 5 durchaus auch unterschiedliche Funktionen erfüllen können. So ist es beispielsweise möglich, eine der Trägerschichten 5 in Form eines Gewebes auszubilden um damit eventuell auftretende Zugkräfte besser aufnehmen zu können und in der Folge einer Zerstörung des Prophylaxeartikels entgegenzuwirken. Eine derartige Gewebeschicht kann z. B. zwischen zwei vollflächigen Trägerschichten 5 eingebettet sein.

Die Trägerschicht 5 weist eine äußere Oberfläche 6 und eine innere Oberfläche 7 auf, wobei die äußere Oberfläche 6 der Trägerschicht 5 die Außenseite des Prophylaxeartikels 1 bzw. jeweils eine Nachbarschicht zur inneren Oberfläche 7 der nächstgelegenen Trägerschicht 5 bildet. Die innere Oberfläche 7 der Trägerschicht 5 bildet entweder die Grenzfläche zur äußeren Oberfläche 6 der nächstgelegenen Trägerschicht 5 bzw. die Grenzfläche zu einer äußeren Oberfläche 8 der Gleitschicht 4. Die Gleitschicht 4 ihrerseits weist eine äußere Oberfläche 8 und eine inneren Oberfläche 9 auf, wobei die innere Oberfläche 9 der Gleitschicht 4 bei Verwendung des Prophylaxeartikels 1 der Haut des Trägers zugewandt ist.

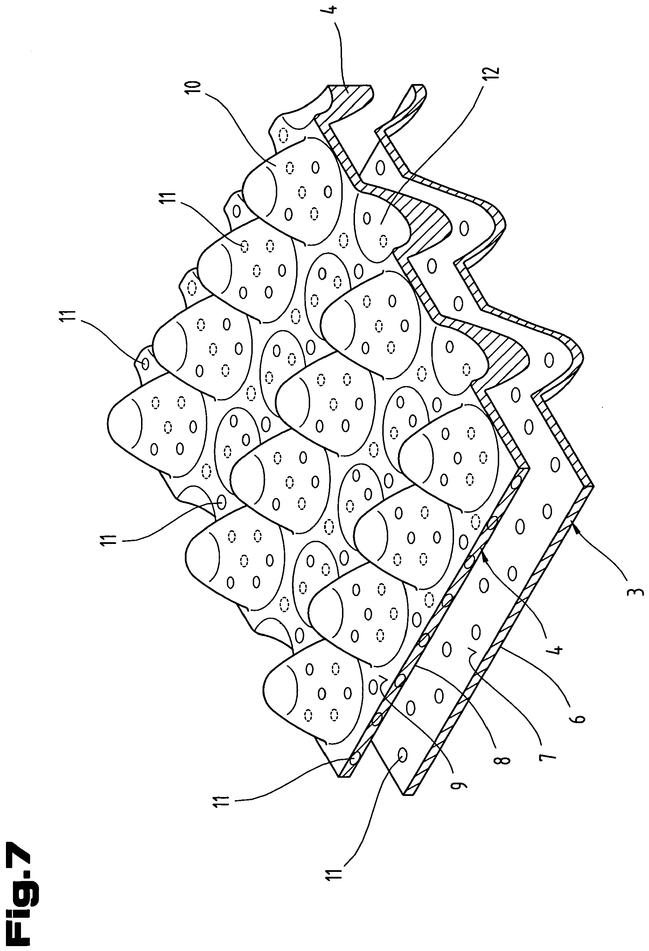

Die Oberflächen 6, 7 der (einzelnen) Trägerschicht(en) 5 bzw. die Oberflächen 8, 9 der Gleitschicht 4 können entweder glatt ausgebildet sein, wie in Fig. 2 dargestellt, wobei bei glatter Ausbildung der Oberfläche 8, 9 die erfindungsgemäße Oberflächenrauhigkeit durch partikulär verkapselte Wirkstoffe und/oder Farbstoffe erzielt wird, wie dies im folgenden noch näher dargestellt wird, oder Gestaltsabweichungen 10 (siehe Fig. 7) aufweisen, wodurch ebenfalls eine Oberflächenrauhigkeit ausgebildet wird und somit die Anziehbarkeit und Nassschlüpfrigkeit des Prophylaxeartikels 1 verbessert wird. Diese Oberflächen mit Gestaltsabweichungen 10 können gemäß einem Herstellungsverfahren nach der EP 0 856 294 B1 hergestellt werden bzw. gemäß der Ausführung nach einem Gegenstand der EP 0 856 294 B1 ausgebildet sein.

Mehrschichtige Prophylaxeartikel 1 aus einem elastomeren Trägermaterial 3, wie z.B. Handschuhe 2, werden vorzugsweise durch Tauchen einer aus dem Stand der Technik bekannten Form in ein das Elastomer enthaltendes Bad erhalten, wie dies bereits aus dem vorgenannten Dokument bekannt ist. Nähere Ausführungen dazu erübrigen sich daher.

Das Elastomer kann sowohl auf Natur- als auch auf Syntheselatex basieren. Von den natürlichen und synthetischen Latices werden vorzugsweise Naturkautschuk, Polychloropren, synthetisches Polyisopren, Nitrilbutadien- und Styrolbutadienkautschuk bzw. eine Mischung dieser Polymere verwendet. Die verwendeten Elastomere können sowohl unvernetzt als auch im vorvernetzten Zustand vorliegen.

Selbstverständlich können alle dem Fachmann aus dem Stand der Technik bekannten Herstellungsverfahren für die Herstellung des Trägermaterials 3 verwendet werden.

Es ist seit langem in der Fachwelt bekannt, dass, um reproduzierbare Schichten des Trägermaterials 3 aus Elastomeren auf einer Form herstellen zu können, auf die Form, beispielsweise auf eine mit entsprechenden Aufrauhungen oder mit einer glatten Oberfläche versehene Keramikform, ein Koagulant aufgebracht wird. Dazu wird üblicherweise die Form in ein Becken bzw. einen Behälter, in welchem der Koagulant in flüssiger Form vorhanden ist, eingetaucht. Dieser Koagulant kann jede aus dem Stand der Technik bekannte Zusammensetzung aufweisen, wie beispielsweise Alkohollösungen von Kalziumsalzen oder dgl. Der Koagulant kann auch ein Trennmittel enthalten, wie beispielsweise Talkum oder Kalziumcarbonat, das, wenn es säurelöslich ist, bei nachfolgenden Säurebehandlungen aus den Oberflächenschichten herausgelöst werden kann, sodass ein sog. puderfreier Handschuh entsteht. Anschließend wird der Koagulant getrocknet.

Daraufhin wird die Form mit dem bevorzugt getrockneten Koagulant in einen Behälter, in dem das Elastomer als Dispersion bzw. Flüssigkeit vorrätig gehalten wird, eingetaucht. Um die Schichtdicke zu erhöhen, kann nach einem kurzen Antrocknen der Latex-Schicht die Form mehrmals eingetaucht werden, sodass eine durchschnittliche Schichtdicke von z.B. 100 µm bis 300 µm erreicht wird. Dieser Tauchvorgang kann erforderlichenfalls (mehrmals) wiederholt werden.

Durch die chemische Reaktion des Elastomers mit dem Koagulant härtet das flüssig aufgetragene Elastomer aus. Bevorzugt wird unmittelbar nach dem Aufbringen des Elstomers auf die Form diese mit Heißluft kurz angetrocknet, sodass die Oberflächen 6, 7 der Trägerschicht 3 fest bzw. geliert werden, wobei diese beispielsweise in einem Wärmeofen oder einem Behälter, in dem Warmluft mit einer Temperatur zwischen 70°C und 140°C hindurchgeführt wird, 15 sec. bis 60 sec. getrocknet werden.

Nach der Zwischentrocknung wird auf die Trägerschicht 3 zumindest in einem Teilbereich der Oberfläche 7 der Trägerschicht 3, vorzugsweise auf die gesamte Fläche, die Gleitschicht 4 aus polymeren Material enthaltend Partikel 11 durch Tauchen oder Sprühen in einem oder mehreren Schritten auf die angetrocknete Trägerschicht 3 aufgetragen. Der Durchmesser der Partikel 11 kann aus einem Bereich mit einer oberen Grenze von 500 µm, insbesondere 400 µm, vorzugsweise 300 µm und einer unteren Grenze von 10 µm, vorzugsweise 30 µm, insbesondere 40 µm, ausgewählt sein. Der Durchmesser der Partikel 11 kann auch einen Wert annehmen, ausgewählt aus einem Bereich mit einer oberen Grenze von 250 µm, vorzugsweise 200 µm, insbesondere 150 µm, und einer unteren Grenze von 50 µm, vorzugsweise 80 µm, insbesondere 100 µm.

Die Schichtdicke der Gleitschicht 4 kann entsprechend den unterschiedlichen Erfordernissen, insbesondere der danach gewünschten Rautiefe und der Größe der Partikel 11, festgelegt werden und beträgt zwischen 2 µm und 300 µm, vorzugsweise 5 µm bis 100 µm, insbesondere 10 µm bis 50 µm. Das Material für die Gleitschicht 4 kann auch solange aufgetragen werden, bis diese eine Dicke, ausgewählt aus einem Bereich mit einer unteren Grenze von 30 µm, vorzugsweise 40 µm, insbesondere 50 µm, und einer oberen Grenze von 500 µm, vorzugsweise 400 µm, insbesondere 300 µm, aufweist bzw. die Gleitschicht einen Dickenwert annimmt, der ausgewählt ist, aus einem Bereich mit einer unteren Grenze von 55 µm, vorzugsweise 60 µm, insbesondere 75 µm, und einer oberen Grenze von 200 µm, vorzugsweise 150 µm, insbesondere 110 µm. Der Durchmesser der Partikel 11 ist vorzugsweise so ausgewählt, dass zumindest die innere Oberflächenrauheit des Prophylaxeartikels 1 erzeugt bzw. verstärkt wird. Vorteilhaft ist es wenn der Partikeldurchmesser zumindest 80 % der Dicke der Gleitschicht 4 beträgt und/oder der Dicke der Gleitschicht 4 entspricht. Es ist aber auch möglich dass der Durchmesser der Partikel 11 größer als die Dicke der Gleitschicht 4 ist.

Die Gleitschicht 4 kann durch ein heterogenes Gemisch aus zumindest einem polymeren Material, wie beispielsweise einer wässrigen Polyurethandispersion, und Partikeln 11, insbesondere Mikrokapseln, Liposomen, etc., gebildet werden.

Als polymeres Material kann je nach Einsatzzweck ein Polyacrylat, ein Polysiloxan, ein Poly(meth)acrylat, ein carboxyliertes Styrol-Butadien Copolymer, ein Polyvinylpyrollidon, ein kationisches Polyurethan und Mischungen daraus, z.B. mit einem Molekulargewicht von mindestens 100.000 Da, ebenso verwendet werden, wie die nichtionischen oder anionischen Ausführungen dieser vorgenannten Materialien. Das heterogene Gemisch, insbesondere die wässrige Dispersion aus den polymeren Materialien und Partikeln 11 und beliebige Mischungen davon, bildet Schichten bzw. Filme mit guten mechanischen Grundeigenschaften und weisen diese vorzugsweise ähnliche Dehnungseigenschaften auf wie die Trägerschicht 3. Die Konzentration der Partikel 11 im heterogenen Gemisch kann aus einem Bereich mit einer unteren Grenze von 1 Gew%, insbesondere 2 Gew%, vorzugsweise 5 Gew%, und einer oberen Grenze von 50 Gew%, vorzugsweise 40 Gew%, insbesondere 30 Gew%, ausgewählt sein. Vorzugsweise ist die Konzentration der Partikel 11 im heterogenen Gemisch aus einem Bereich mit einer unteren Grenze von 6 Gew%, vorzugsweise 7 Gew%, insbesondere 10 Gew% und einer oberen Grenze von 25 Gew%, vorzugsweise 20 Gew%, insbesondere 15 Gew%, ausgewählt.

Für die Verwendung der Partikel 11, insbesondere Mikrokapseln, zum Aufsprühen auf das Trägermaterial 3, insbesondere auf die Trägerschicht 5, und/oder die Gleitschicht 4 können diese wasserlösliche Eigenschaften aufweisen, sodass beim Tragen des Prophylaxeartikels 1 bereits durch den Kontakt der Partikel 11 mit dem Schweiß der Haut des Verwenders die Wirkstoffe und/oder Farbstoffe durch das zumindest partielle Auflösen des Hüllmaterials freigesetzt werden.

Beim Aufbringen der Partikel 11 auf die Trägerschicht 5 bzw. Gleitschicht 4 mittels Tauchverfahren weisen diese bevorzugt wasserunlösliche Eigenschaften auf, um nicht die Wirkstoffe und/oder Farbstoffe bereits während des Herstellungsverfahrens insbesondere während der diversen Waschverfahren freizusetzen. Es ist hierbei von Vorteil, dass die Partikel 11 unter mechanischer Beanspruchung, wie z.B. Druck- oder Reibbelastung, beim Tragen und insbesondere beim Anziehen des Prophylaxeartikels 1 zum Zerbersten veranlasst und somit die Wirkstoffe und/oder Farbstoffe freigesetzt werden.

Die Partikel 11, insbesondere Mikrokapseln, beinhalten Wirkstoffe und/oder Farbstoffe. Die in den Partikeln 11 enthaltenen Farbstoffe können eine andere Farbe als die Gleitschicht 4 bzw. das Trägermaterial 3 bzw. die Trägerschicht 5 in oder auf welcher die Partikel 11 aufgebracht sind, aufweisen auf um das Zerbersten der Partikel 11 auch visuell beobachten zu können.

Die Partikel 11, insbesondere Mikrokapseln, können auch Wirkstoffe mit antibakterieller Wirkung beinhalten, ausgewählt aus einer Gruppe umfassend, Ammoniumiodid, Chlorhexidin, Chlorhexidindiacetat, Chlorhexidindigluconat, Chlorhexidindichlorid, Hexamidindiisothionat, Hexetidin, Lauralkoniumbromid, Aloe Vera, Lauralkoniumchlorid, Laurtrimoniumchlorid, Laurylpyridiniumchlorid, Orangenhautextrakt, Quaternium 73, Benzalkoniumchlorid, Bromchlorophen, 2-brom-2-Nitropropan-1,3-Propaniol, Captan, Cetyldiamoniumbromid, Cetylpyridiniumchlorid, Chlorothymol, Chloroxylenol, Kupfer PCA, Dichlorbenzylalkohol, Nonoxynol-9, etc. Zur Überprüfung der antimikrobiellen Wirkung des Prophylaxeartikels 1 können verschiedene Testmethoden, wie z.B. die Agar-Diffusionsmethode, die Keimzählmethode, etc., durchgeführt werden.

In einer alternativen Ausführungsform beinhalten die Mikrokapseln antitranspirante Wirkstoffe insbesondere ausgewählt aus einer Gruppe umfassend Aluminiumglycinat, Aluminiumchlorhydratglycinat, Aluminiumzirconiumtetrachlorhydroxyglycin, Allantoinderivate, wie z.B. Alcloxa, Aldioxa, Aluminiumchlorid, Aluminiumchlorhydrex, Aluminium PCA, Zirconiumchlorhydrat und Aluminiumchlorhydrat, etc.

In einer alternativen Ausführungsform beinhalten die Mikrokapseln antivirale und/oder antimykotische und/oder spermizide Wirkstoffe, welche dem Fachmann aus der Fachliteratur bekannt sind, und daher an dieser Stelle keiner weiteren Erwähnung bedürfen.

Weiters können in den Partikeln 11 pflegende Wirkstoffe wie z.B. Glycerylstearat, Glyceryllaurat, Octylstearat, Octylpalmitat, Tocopherylnicotinat, PEG, Kollagen. Fruchtsäuren, Fettsäuren, Quercetin, etc., enthalten sein.

In den Mikrokapseln können auch Vitamine ausgewählt aus einer Gruppe umfassend Verbindungen mit Retinoidstruktur (Vitamine A), Vitamin-B-Komplex, Ascorbinsäuren (Vitamine C), Calciferole (Vitamine D), Tocopherole (Vitamine E), Vitamine K, Flavanoide und Biotin, Spurenelemente, Mineralstoffe, Pflanzeninhaltsstoffe, insbesondere sekundäre Pflanzeninhaltsstoffe, enthalten sein. Es werden bevorzugt natürliche Verbindungen der Vitamine in den Partikeln 11 verkapselt. Allerdings können selbstverständlich auch synthetische Verbindungen von Vitaminen eingesetzt werden.

Es können selbstverständlich auch Partikel 11 mit Wirkstoffen unterschiedlicher Wirkung auf den selben Prophylaxeartikel 1 aufgebracht sein, insbesondere in der Gleitschicht 4.

Die Konzentration des zumindest einen Wirkstoffes und/oder Farbstoffes im Partikel 11 ist aus einem Bereich mit einer unteren Grenze von 1%, vorzugsweise 2 %, insbesondere 5 %, und einer oberen Grenze von 10 %, vorzugsweise 15 %, insbesondere 20 %, ausgewählt.

Selbstverständlich kann die Gleitschicht 4 auch aus einem Gemisch von mehreren unterschiedlichen polymeren Materialien in einer wässerigen Dispersion gebildet werden. So wird bevorzugt die Dispersion durch ein Gemisch aus 0 Gew% bis 30 Gew% Polyurethan-, 1 Gew% bis 40 Gew% Polyacrylat-, 1 Gew% bis 20 Gew% Polysiloxandispersion und 0 Gew% bis 10 Gew% Füllstoffe und der restliche Anteil aus Wasser gebildet. Als Füllstoffe können pulver- oder puderförmige Materialien, wie z.B. Kreide, Kalk, Gips, Siliciumdioxid und/oder Maisstärke, zur Anwendung kommen. Für die Herstellung der Gleitschicht 4 beim erfindungsgemäßen Prophylaxeartikel 1, insbesondere einem Handschuh 2, können aber auch andere Mischungen, wie beispielsweise 5 Gew% bis 15 Gew% Polyurethan-, 1 Gew% bis 8 Gew% Polyacrylat-, 2 Gew% bis 6 Gew% Polysiloxandispersion und 4 Gew% bis 6 Gew% Füllstoffe und der Rest Wasser ebenso wie die nachfolgend in den einzelnen Klammern angegebenen Mischungsverhältnisse, bei welchen die einzelnen Anteile an Gew% der Dispersionen aus Polyurethan, Polyacrylat, Polysiloxan und Füllstoffe durch Schrägstriche getrennt sind, wobei der restliche Anteil auf 100 Gew% durch Hinzumischung von Wasser gebildet ist, verwendet werden. Der Trockengehalt der Dispersionen ist vom Fachmann festlegbar, wobei der Feststoffgehalt bei Polyurethan zwischen 30 % und 50 %, bei Polyacrylat 30 % bis 50 % und bei Polysiloxan 20 % bis 40 % betragen kann. Bei diesen Gemischen handelt es sich um folgende (2-7/4-10/3-12/0-5), (0-10/2-6/3-10/3-7), (8-18/5-15/4-7/5-10), (12-22/12-26/16-20/0-4), (17-26/18-32/10-14/2-6), (24-30/28-40/15-20/6-9), (24-30/25-35/5-10/3-7), (21-27/4-9/1-4/6-8), (21-27/12-22/3-9/4-7), (21-27/28-36/12-20/2-7), (9-12/1-3/2-6/5-9), (12-22/4-9/1-4/0-3), (17-26/5-11/7-10/0-4), (2-7/12-22/14-20/3-8), (5-15/28-36/14-20/5-10), (0-10/24-29/9-16/5-8).

An dieser Stelle sei der Ordnung halber erwähnt, dass es selbstverständlich möglich ist, das polymere Material der Gleitschicht 4 bzw. natürlich auch das Elastomer bzw. die Koagulationslösung durch Zusatz eines oder mehrerer dem Fachmann aus dem bekannten Stand der Technik in unterschiedlichen Ausführungen bekannten Viskositätsregler in ihrer Viskosität an die gewünschten Eigenschaften anzupassen. Dadurch ist es möglich, die Viskosität der üblicher Weise als Dispersionen vorliegenden Produkte bzw. Materialien auf die jeweils gewünschten Einsatzzwecke anzupassen, um entsprechende Schichtstärken beim Tauchen oder eine gleichmäßige Ablagerung der verschiedenen Materialien an einer Oberfläche der Tauchformen zu erreichen.

Die Partikel 11 können palmar und/oder dorsal in zumindest einem Teilbereich des Prophylaxeartikels 1, wie im Bereich des distalen Unterarms, der Handwurzelknochen, der Mittelhandknochen, der Grund-, Mittel- und Endglieder der Finger, aufgebracht sein. Es sind zwischen 40 % und 90 %, vorzugsweise 50 % bis 80 %, insbesondere 60 % und 70 %, der Teilbereiche des Prophylaxeartikels 1 durch Partikeln 11, insbesondere Mikrokapseln, bedeckt.

Die Angabe der Teilbereiche bezieht sich nicht nur auf die Trägerschicht 3, sondern auch auf die Gleitschicht 4.

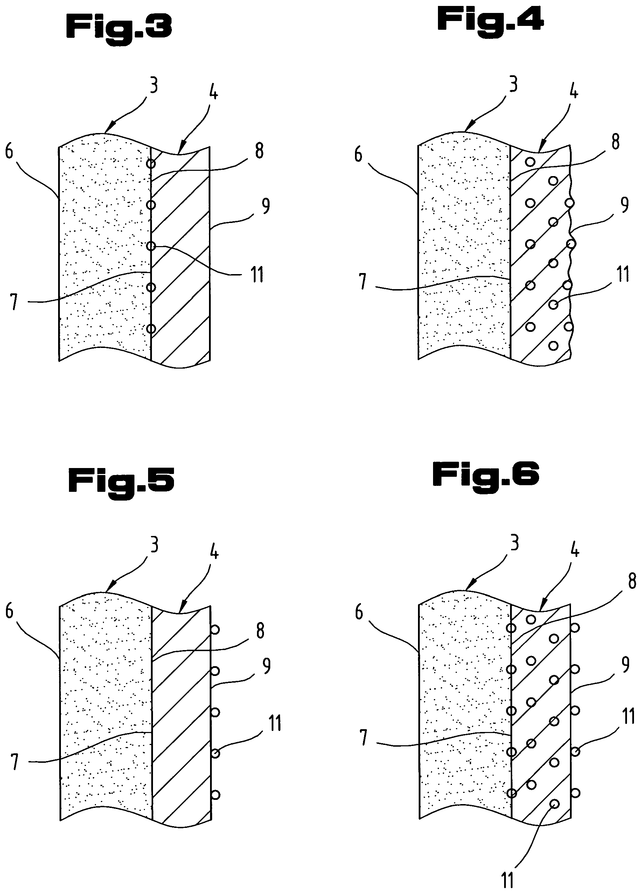

In den Fig. 3 bis 6 werden verschiedenste Ausführungsvarianten für die Anordnung der Partikel 11 beinhaltend die Wirkstoffe und/oder Farbstoffe im Prophylaxeartikel 1 dargestellt.

Fig. 3 stellt eine Anordnung der Partikel 11 auf der inneren Oberfläche 7 der Trägerschicht 5 bzw. zwischen der inneren Oberfläche 7 der Trägerschicht 5 und der äußeren Oberfläche 8 der Gleitschicht 4 dar. Die Partikel 11 können mittels Tauchen einer Form in eine wässrige Dispersion mit Partikeln 11 auf die innere Oberfläche 7 der Trägerschicht 5 aufgebracht werden.

In einer weiteren Ausführungsvariante gemäß Fig. 4 wird eine Anordnung der Partikel 11 in der Gleitschicht 4 dargestellt, wobei die Partikel 11 mit dem heterogenen Gemisch zur Ausbildung der Gleitschicht 4 aufgebracht werden, sodass die Partikel 11 vorzugsweise homogen in der Gleitschicht 4 verteilt sind.

Fig. 5 zeigt eine Anordnung der Partikel 11 auf der inneren Oberfläche 7 der Gleitschicht 4, die wie in Fig. 3 beschrieben aufgebracht werden.

In Fig. 6 wird die Anordnung der Partikel 11 auf der inneren Oberfläche 7 der Trägerschicht 5 bzw. zwischen der inneren Oberfläche 7 der Trägerschicht 5 und der äußeren Oberfläche 8 der Gleitschicht 4 bzw. in der Gleitschicht 4 bzw. auf der inneren Oberfläche 9 der Gleitschicht 4 dargestellt, wobei die Partikel durch mehrmaliges Eintauchen in Dispersionslösungen aufgebracht werden.

Durch die Darstellung der unterschiedlichsten Ausführungsvarianten soll für den Fachmann dargelegt werden, dass er entsprechend der technischen Lehre der Erfindung, die Partikel 11 je nach den Erfordernissen an den unterschiedlichsten Stellen des Prophylaxeartikels 1 anordnen kann.

Fig. 7 zeigt in Explosionsdarstellung eine weitere Ausführungsvariante des Prophylaxeartikels 1, in Detailansicht, wonach die Partikel 11 auch auf Gestaltsabweichungen 10, z.B. Erhebungen bzw. Vertiefungen, des Prophylaxeartikels 1 angeordnet sein können.

Nach dem Herausheben des Formträgers mit den Tauchformen aus dem heterogenen Gemisch aus polymerem Material und Mikrokapseln zur Herstellung der Gleitschicht 4, wird die aufgebrachte Gleitschicht mit Heißluft, beispielsweise bei einer Temperatur zwischen 70°C und 140°C, bevorzugt 90°C bis 110°C, über eine Dauer von 15 sec. bis 60 sec. angetrocknet. Bevorzugt wird die Temperatur und die Zeitdauer der Heißluftbehandlung so abgestimmt, dass die Oberfläche der Gleitschicht 4 in einen gelartigen bzw. festen Zustand übergeht.

Unmittelbar darauf werden die Tauchformen mit den darauf befindlichen Rohteilen der Prophylaxeartikel 1, insbesondere Handschuhe 2, in ein weiteres Tauchbecken eingetaucht, in welchem die Gleitschicht 4 mit heißem Wasser, mit einer Temperatur zwischen 40°C und 95°C, bevorzugt 70°C bis 90°C, besprüht oder gespült wird.

Durch diese Behandlung mit heißem Wasser wird die chemische Reaktion in der Gleitschicht 4 und des Trägermaterials 3 eingeleitet bzw. unterstützt, sodass es zum Einleiten oder zur vollständigen Koagulation in dieser Schicht kommt.

Bei dem vorstehend beschriebenen Verfahren wird die Rauheit bzw. die gemittelte Rautiefe in der Gleitschicht einerseits dadurch erreicht, dass während bzw. unmittelbar nach der Beaufschlagung der Gleitschicht 4, mit heißem Wasser der Gleitschicht 4 zwischen 40 % bis 70 %, bevorzugt 50 % bis 60 % des Wassergehaltes entzogen werden und andererseits durch die Beimengung von Partikeln 10 zur Gleitschicht 4. In Folge des raschen Wasserentzuges kommt es zum Aufbau relativ hoher Oberflächenspannungen im Bereich der Gleitschicht 4 und führt dies zu einem Einsinken bzw. einer Verringerung der Dicke der Gleitschicht 4 wodurch Kavitäten 12 erzeugt werden können.

Die durch das erfindungsgemäße Verfahren hergestellten Gestaltsabweichungen 10 können beispielsweise einer solchen Tiefe entsprechen, bei welcher die Gestaltsabweichungen 10 so groß sind, dass sie sich über die gesamte Dicke der Gleitschicht 4, d.h. bis auf die innere Oberfläche des Trägermaterials 3 erstrecken.

Im Rahmen der vorliegenden Erfindung ist es aber auch möglich, dass sowohl auf der inneren Oberfläche 7 als auch auf der äußeren Oberfläche 6 des Trägermaterials 3 eine Gleitschicht 4 angeordnet ist, wobei dies dadurch erfolgen kann, dass nach Herstellen des Trägermaterials 3 der Prophylaxegegenstand 1 von der Tauchform abgezogen wird und in gewendeter Lage, also mit der äußeren Oberfläche 6 des Trägermaterials 3 auf der Tauchform anliegend, auf die gleiche oder eine andere Tauchform aufgezogen wird, um danach auch auf die innere Oberfläche 7 des Trägermaterials 3, die nun auf der Tauchbadseite angeordnet ist, ebenfalls eine Gleitschicht 4 aufzubringen.

Durch die Oberflächenstrukturierung in Folge der Gestaltsabweichungen 10, Partikel 11 und Kavitäten 12 werden Bereiche mit verringerter Wandstärke der Gleitschicht 4 gebildet, sodass der Prophylaxeartikel 1, insbesondere der Handschuh 2, nur bereichsweise auf der Oberfläche der menschlichen Haut aufliegt und wird dadurch zum einen die Berührungsfläche mit der Haut verringert, zum anderen dadurch die Schlüpfrigkeit, insbesondere die Nassschlüpfrigkeit erhöht. Gleichzeitig werden dadurch Hohlräume geschaffen, die auch den beim Arbeiten entstehenden Schweiß aufnehmen können, sodass auch der Tragekomfort derartiger Prophylaxegegenstände 1, insbesondere Handschuhe 2, erheblich verbessert wird und aufgrund der partiellen Ansammlung des Schweißes bei wasserlöslichen Hüllen der Partikel 11 ein ausreichender Zeitraum für die Freisetzung der Wirkstoffe der Partikel 11 ermöglicht wird, bevor der Schweiß gegebenenfalls abtransportiert wird.

Obwohl in voranstehender Beschreibung das Aufbringen der Partikel 11 auf die Trägerschicht 5 und/oder Gleitschicht 4 mittels Tauchen bzw. Sprühen dargestellt wurde sind auch andere Verfahren möglich. So kann z.B. durch gegengleiche Aufladung der Oberfläche 7 der Trägerschicht 5 bzw. der Oberfläche 9 der Gleitschicht 4 und der Partikel 11 über elektrostatische Anziehung erreicht werden, insbesondere bei partieller Aufladung besagter Oberflächen 7, 9, dass die Partikel 11 selektiv an die beschriebenen Teilbereiche herangeführt und angelagert werden. Eine Aufladung der Partikel 11 kann z.B. durch Elektronenbeschuss oder durch Wanderung durch ein elektrostatisches Feld erfolgen.

Nach einer weiteren, nicht dargestellten Ausführungsvariante ist es möglich, die Gestaltabweichungen 10, insbesondere die Vertiefungen, mit unregelmäßiger Form, regelmäßig über zumindest einen Teilbereich der Gleitschicht 4 auszubilden, wobei diese unregelmäßige Ausbildung insbesondere wieder durch den raschen Flüssigkeitsentzug aus der Gleitschicht hergestellt werden kann. Es erweist sich dabei von Vorteil, wenn der Anteil der Vertiefungen, welche sich über die gesamte Dicke der Gleitschicht 4 erstrecken, ausgewählt ist aus einem Bereich mit einer unteren Grenze von 20 %, insbesondere 35 %, vorzugsweise 40 %, und einer oberen Grenze von 95 %, insbesondere 80 %, vorzugsweise 75 %, bezogen auf die Gesamtanzahl der Vertiefungen in der Gleitschicht 4 des Prophylaxeartikels 1. Dabei können Vertiefungen entstehen, die kraterförmig sich in Richtung auf die Trägerschicht verjüngend ausgebildet sind, wobei diese vorzugsweise einen maximalen Durchmesser, in Draufsicht gesehen, ausgewählt aus einem Bereich mit einer oberen Grenze von 30 µm, vorzugsweise 25 µm, insbesondere 20 µm, und einer unteren Grenze von einem 1 µm, vorzugsweise 5 µm, insbesondere 10 µm, aufweisen.

Der Ausdruck "maximaler Durchmesser" beschreibt in diesem Zusammenhang jenen Durchmesser, welcher ein die Vertiefung in Draufsicht gesehen umhüllender Kreis aufweist.

Des weiteren sei an dieser Stelle vermerkt, dass mit dem Begriff Vertiefung bzw. Erhebung zum Ausdruck gebracht werden soll, dass, je nach Sichtweise, das die Vertiefung umgebende Material auch als Erhebung gesehen werden kann und umgekehrt, also im Wesentlichen der Bezugspunkt im Hinblick auf die Ausdrücke Vertiefung bzw. Erhebung ein unterschiedlicher in der Gleitschicht 4 ist.

Der rasche Wasserentzug, d.h. Flüssigkeitsentzug, aus der Gleitschicht 4, kann einerseits wie bereits beschrieben erreicht werden oder aber auch durch Verfahrensweisen, bei denen der Flüssigkeitsentzug alternativ oder in Kombination hierzu innerhalb einer Zeitspanne mit einer unteren Grenze von 10 sec., insbesondere 25 sec., vorzugsweise 50 sec., und einer oberen Grenze von 20 min., insbesondere 15 min., vorzugsweise 10 min., erfolgt. Darüber hinaus ist es möglich, den Flüssigkeitsentzug wiederum alternativ oder zusätzlich über die Temperatur zu steuern, in dem die Temperatur während des Flüssigkeitsentzuges einen Wert aufweist, der ausgewählt werden kann aus einem Bereich mit einer unteren Grenze von 60°C, insbesondere 66°C, vorzugsweise 70°C, und einer oberen Grenze von 150°C, insbesondere 125°C, vorzugsweise 110°C. Die erforderliche Temperatur kann mit herkömmlichen Mitteln erreicht werden, in dem z.B. diverse Öfen. wie z.B. Infrarotstrahler oder auch andere Heizquellen. eingesetzt werden. Ebenso ist die bereits beschriebene Heißwassermethode durchführbar.

Bevorzugt wird durch das Zusammenwirken der einzelnen Verfahrensparameter das Verfahren derart durchgeführt, dass die entstehenden Erhebungen zumindest großteils netzförmig mit miteinander verbundenen Stegen ausgebildet werden, wobei sich gemäß einer bevorzugten Ausführungsvariante zumindest ein Teil der Stege mit einer Höhe die einen Wert aufweist, der im Bereich zwischen 25 % und 100 %, vorzugsweise 30 % und 75 %, insbesondere 40 % und 60 %, der Gesamtdicke der Gleitschicht beträgt, gebildet werden. Durch eine derartige Ausbildung einer netzartigen Struktur, wie sie ansatzweise bereits in der bereits zitierten EP 0 856 294 A1 der Anmelderin beschrieben wurden, deren Inhalt Teil gegenständlicher Beschreibung ist, wird eine entsprechende Verringerung der Kontaktfläche zwischen dem Prophylaxeartikel 1, insbesondere der Gleitschicht 4, und der Haut des Verwenders erreicht, wodurch wiederum eine entsprechend verbesserte Anziehbarkeit des Prophylaxeartikels 1 und in der Folge auch eine entsprechend verbesserte Ausziehbarkeit desselben erreicht werden kann. Dieser Effekt kann insofern verstärkt werden, in dem eben beschriebene Ausführungsvariante der Erfindung mit jenen Ausführungsvarianten, welche Partikeln 11 zur Erzielung einer gewissen Oberflächenrauhigkeit verwenden, kombiniert werden, wobei hier wiederum das Hervortreten der Partikel 11 über die Gleitschicht 4 den Effekt der verbesserten Anziehbarkeit erhöht, zugleich aber durch diese Kombination in Folge der Partikel 11 wiederum ermöglicht werden kann, dass die zu applizierenden Wirkstoffe bzw. Farbstoffe, insbesondere bei Verwendung eines wasserlöslichen bzw. druckempfindlichen Hüllmaterials für die Partikel 11, diese bereits während des Anziehens ihren Inhalt, also die Wirk- bzw. Farbstoffe, frei setzen und somit durch das Überziehen des Prophylaxeartikels 1 z.B. über die Hand diese Wirk- bzw. Farbstoffe zumindest annähernd gleichmäßig über die Haut verteilt werden und somit eine Verbesserung gegenüber der Ausführungsvariante, bei der die Wirkstoffe erst durch den Flüssigtransport über die Poren der Gleitschicht 4 an die Haut abgegeben werden, erzielt wird, sodass durch dieses gleichmäßige "Verstreichen" der Wirk- bzw. Farbstoffe über die Haut diese großflächig und insbesondere unmittelbar beim ersten Verwenden des Prophylaxeartikels über die Haut verteilt werden.

Erklärend sei angemerkt, dass mit dem Ausdruck "druckempfindlich" in bezug auf das Hüllmaterial der Partikel 11 gemeint ist, dass diese bei Druckbeaufschlagung, z.B. in Folge des Anziehvorganges oder aber auch durch eine entsprechende Reibbelastung bersten und dadurch die Wirkstoffe bzw. Farbstoffe freigesetzt werden.

Als vorteilhaft erweist es sich auch, wenn ein Wirkstoff und/oder Farbstoff verwendet wird, der in Wasser eine Löslichkeit bei 20°C aufweist, die ausgewählt ist aus einem Bereich mit einer unteren Grenze von 1 g/l, vorzugsweise 3 g/l, insbesondere 4,5 g/l, und einer oberen Grenze von 20 g/l, vorzugsweise 15 g/l, insbesondere 8 g/l. Dadurch wird alternativ oder in Kombination dazu, dass der Wirk- und/oder Farbstoff in einer Menge eingesetzt wird, die eine Abgabe von vorzugsweise zumindest annähernd gleichmäßigen Dosen dieser Wirk- und/ oder Farbstoffe während der Tragedauer des Prophylaxeartikels 1, erreicht, dass zum Einen eine in bezug auf die Zeit gesehene partielle Überdosierung vermieden wird und zum Anderen die Abgabe der Wirk- bzw. Farbstoffe über die gesamte Tragedauer gewährleistet werden kann.

Hinsichtlich der Hautverträglichkeit erweist es sich weiters als Vorteil, wenn Wirk- bzw. Farbstoffe Verwendung finden, die entweder selbst oder eine damit hergestellte Lösung einen pH-Wert ausgewählt aus einen Bereich von 5,5 bis 7,5 aufweist. Vorzugsweise werden dabei Wirkstoffe ausgewählt, die ein neutrales bzw. leicht saures Milieu ergeben, wobei geringfügig basische Lösungen aus dermatologischer Sicht auch noch bedenkenlos verwendbar sind.

Abschließend sei zur Vollständigkeit darauf hingewiesen, dass, wie bereits in der EP 0 856 294 A beschrieben, vorzugsweise ein Elastomer verwendet wird, welches bereits vernetzt oder größtenteils vorvernetzt ist, wobei unvernetzte Elastomere selbstverständlich auch einsetzbar sind. Dieses Elastomer wird gemäß einer Ausführungsvariante auf eine Tauchform, welches die Gestalt des Endproduktes vorgibt, aufgebracht und koaguliert und erzeugt so eine den jeweiligen Bedürfnissen angepasste, mehr oder weniger dünne Schicht aus dem entstehenden elastischen Material des Elastomer. Das Tauchbad selbst enthält die üblichen Compoundierungszutaten, wie beispielsweise Schwefel, Zinkoxid, organische Beschleuniger (u.a. Zinksalze von Dithiocarbamaten, Thiurame, Thioharnstoff, etc.), Stabilisatoren, Wachse, Alterungsschutzmittel, Viskositätsregler, Füllstoffe, Farben, usw.

Der Ordnung halber sei an dieser Stelle auch erwähnt, dass in der vorliegenden Beschreibung immer wieder der Begriff "Elastomer" durchgehend verwendet wurde, unabhängig davon, ob es sich um eine Dispersion oder die ausgehärtete, trockene, vernetzte Schicht oder die feste Phase dieses Materials handelt, auch wenn diese wie z.B. bei Naturlatex üblicherweise als Kautschuk bezeichnet wird. Es wäre daher auch möglich, die aus den einzelnen vorgenannten Materialien gebildeten Schichten als Kautschukschichten zu bezeichnen. Dieses polymere Material kann entsprechend den vorstehenden Angaben zusammengesetzt sein.

Die Ausführungsbeispiele zeigen mögliche Ausführungsvarianten des Prophylaxeartikels 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt. Es sind also auch sämtliche denkbaren Ausführungsvarianten, die durch Kombinationen einzelner Details der dargestellten und beschriebenen Ausführungsvariante möglich sind, vom Schutzumfang mitumfasst, und daher diese nicht mehrfach beschrieben.

Der Ordnung halber sei abschließend daraufhingewiesen, dass zum besseren Verständnis des Aufbaus der Prophylaxeartikel 1, dieser bzw. dessen Bestandteile teilweise unmaßstäblich und/oder vergrößert und/oder verkleinert dargestellt wurden.

Die den eigenständigen erfinderischen Lösungen zugrundeliegende Aufgabe kann der Beschreibung entnommen werden.

Vor allem können die einzelnen in den Fig. 1, 2, 3; 4; 5; 6 ;7 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

### Bezugszeichenaufstellung

- 1: Prophylaxeartikel
- 2: Handschuh
- 3: Trägermaterial
- 4: Gleitschicht
- 5: Trägerschicht

- 6: äußere Oberfläche
- 7: innere Oberfläche
- 8: äußere Oberfläche
- 9: innere Oberfläche
- 10: Gestaltsabweichung

- 11: Partikel
- 12: Kavitäten

## Patentansprüche

1. Mehrschichtiger Prophylaxeartikel (1), insbesondere ein medizinischer Handschuh (2), aus einer elastomeren Trägerschicht (5), wie z.B. einem Synthese- oder Naturlatex, mit einer inneren und einer äußeren Oberfläche (6, 7), mit zumindest einem Wirkstoff innerhalb von Partikeln (11), insbesondere Mikrokapseln, **dadurch gekennzeichnet, dass** auf der inneren Oberfläche (7) zumindest in einem Teilbereich eine Gleitschicht (4) aus einem polymeren Material mit einer inneren (9) und einer, der inneren Oberfläche (7) der Trägerschicht (5) zugewandten, äußeren Oberfläche (8) angeordnet ist, wobei auf der inneren Oberfläche (9) der Trägerschicht (5) und/oder zwischen der Trägerschicht (5) und der Gleitschicht (4) und/oder in der Gleitschicht (4) und/oder auf der inneren Oberfläche (9) der Gleitschicht (4) zumindest im Teilbereich Partikel (11) mit zumindest einem Wirkstoff und/oder Farbstoff mit einem maximalen Durchmesser, ausgewählt aus einem Bereich mit einer oberen Grenzen von 500 µm, insbesondere 400 µm, vorzugsweise 300 µm und einer unteren Grenze von 10 µm, vorzugsweise 30 µm, insbesondere 40 µm, angeordnet sind und die Gleitschicht (4) regelmäßig wiederkehrende Erhöhungen bzw. Vertiefungen mit unregelmäßiger Form, hergestellt durch raschen Flüssigkeitsentzug aus der Gleitschicht (4), aufweist, wobei sich ein Anteil der Vertiefungen, ausgewählt aus einem Bereich mit einer unteren Grenze von 20 %, insbesondere 35 %, vorzugsweise 40 %, und einer oberen Grenze von 95 %, insbesondere 80 % vorzugsweise 75 %, bezogen auf die Gesamtzahl der Vertiefungen, über die gesamte Dicke der Gleitschicht (4) erstreckt.

2. Prophylaxeartikel (1) nach Anspruch 1, **dadurch gekennzeichnet, dass** der Durchmesser der Partikel (11) aus einem Bereich mit einer oberen Grenze von 250 µm, vorzugsweise 200 µm, insbesondere 150 µm, und einer unteren Grenze von 50 µm, vorzugsweise 80 µm, insbesondere 100 µm, ausgewählt ist.

3. Prophylaxeartikel (1) nach Anspruch 1 oder 2, **dadurch gekennzeichnet, dass** der Durchmesser der Partikel (11) zumindest 80 %, vorzugsweise zumindest 85%, insbesondere zumindest 90 %, der Dicke der Gleitschicht (4) beträgt.

4. Prophylaxeartikel (1) nach Anspruch 3, **dadurch gekennzeichnet, dass** der Durchmesser der Partikel (11) gleich groß ist wie die Dicke der Gleitschicht (4).

5. Prophylaxeartikel (1) nach Anspruch 3, **dadurch gekennzeichnet, dass** der Durchmesser der Partikel (11) größer ist als die Dicke der Gleitschicht (4).

6. Prophylaxeartikel (1) nach einem der Ansprüche 1 bis 5, **dadurch gekennzeichnet, dass** der Teilbereich den Bereich des distalen Unterarms und/oder der Handwurzelknochen und/oder der Mittelhandknochen und/oder der Grund-, Mittel- und Endglieder der Finger, umfasst.

7. Prophylaxeartikel (1) nach einem der Ansprüche 1 bis 6, **dadurch gekennzeichnet, dass** die Partikel (11) und/oder die Schicht sowohl palmar als auch dorsal in zumindest einem Teilbereich aufgebracht sind.

8. Prophylaxeartikel (1) nach einem der Ansprüche 1 bis 7, **dadurch gekennzeichnet, dass** sich der Teilbereich über einen Bereich der inneren Oberfläche (7) der Trägerschicht (5) und/oder zwischen der Trägerschicht (5) und der Gleitschicht (4) und/oder in der Gleitschicht (4) und/oder auf der inneren Oberfläche (9) der Gleitschicht (4) mit einer unteren Grenze von 40 %, vorzugsweise 50 %, insbesondere 60 %, und einer oberen Grenze von 100 %, vorzugsweise 80 %, insbesondere 70 %, erstreckt.

9. Prophylaxeartikel (1) nach einem der Ansprüche 1 bis 8, **dadurch gekennzeichnet, dass** die Partikel (11) und/oder die Schicht eine andere Farbe als die Träger- (5) bzw. Gleitschicht (4) aufweisen.

10. Prophylaxeartikel (1) nach einem der Ansprüche 1 bis 9, **dadurch gekennzeichnet, dass** die Partikel (11) wasserunlöslich sind.

11. Prophylaxeartikel (1) nach einem der Ansprüche 1 bis 9, **dadurch gekennzeichnet, dass** die Partikel (11) wasserlöslich sind.

12. Prophylaxeartikel (1) nach einem der Ansprüche 1 bis 11, **dadurch gekennzeichnet, dass** der Wirkstoff antibakterielle bzw. antivirale bzw. antimykotische bzw. antitranspirante bzw. spermizide bzw. pflegende Wirkung aufweist.

13. Prophylaxeartikel (1) nach Anspruch 12, **dadurch gekennzeichnet, dass** der Wirkstoff ausgewählt ist aus einer Gruppe, umfassend Chlorhexidin, z.B. ein Gluconat, ein Acetat, ein Hydrochlorid, Nonoxinol 9 und Aloe Vera.

14. Prophylaxeartikel (1) nach einem der Ansprüche 1 bis 13, **dadurch gekennzeichnet, dass** der Wirkstoff aus einer Gruppe, umfassend Vitamine, Pflanzeninhaltsstoffe, insbesondere sekundäre Pflanzeninhaltsstoffe, ausgewählt sind.

15. Prophylaxeartikel (1) nach Anspruch 14, **dadurch gekennzeichnet, dass** Vitamine aus einer Gruppe umfassend Verbindungen mit Retinoidstruktur (Vitamine A), Vitamin-B-Komplex, Ascorbinsäure (Vitamine C), Calciferole (Vitamine D), Tocopherole (Vitamine E), Vitamine K, Flavanoide und Biotin ausgewählt sind.

16. Prophylaxeartikel (1) nach einem der Ansprüche 12 bis 15, **dadurch gekennzeichnet, dass** die Konzentration des zumindest einen Wirkstoffes und/oder Farbstoffes im Partikel (11) aus einem Bereich mit einer unteren Grenze von 1 %, vorzugsweise 2 %, insbesondere 5 %, und einer oberen Grenze von 20 %, vorzugsweise 15 %, insbesondere 10 %, ausgewählt ist.

17. Prophylaxeartikel (1) nach einem der Ansprüche 1 bis 16, **dadurch gekennzeichnet, dass** eine Hülle der Partikel (11) druckempfindlich ist.

18. Prophylaxeartikel (1) nach einem der Ansprüche 1 bis 17, **dadurch gekennzeichnet, dass** die Partikel (11) in dem zumindest einen Teilbereich die Gleitschicht (4) bilden.

19. Prophylaxeartikel (1) nach einem der Ansprüche 1 bis 18, **dadurch gekennzeichnet, dass** eine Dicke der Gleitschicht (4) ausgewählt ist aus einem Bereich mit einer unteren Grenze von 30 µm, vorzugsweise 40 µm, insbesondere 50 µm, und einer oberen Grenze von 500 µm, vorzugsweise 400 µm, insbesondere 300 µm.

20. Prophylaxeartikel (1) nach Anspruch 19, **dadurch gekennzeichnet, dass** die Dicke der Gleitschicht (4) ausgewählt ist aus einem Bereich mit einer unteren Grenze von 55 µm, vorzugsweise 60 µm, insbesondere 75 µm, und einer oberen Grenze von 200 µm, vorzugsweise 150 µm, insbesondere 110 µm.

21. Prophylaxeartikel (1) nach einem der Ansprüche 1 bis 20, **dadurch gekennzeichnet, dass** die Vertiefungen einen maximalen Durchmesser, in Draufsicht gesehen, ausgewählt aus einem Bereich mit einer oberen Grenze von 30 µm, vorzugsweise 25 µm, insbesondere 20 µm, und einer unteren Grenze von 1 µm, vorzugsweise 5 µm, insbesondere 10 µm, aufweisen.

22. Prophylaxeartikel (1) nach einem der Ansprüche 1 bis 21, **dadurch gekennzeichnet, dass** die Vertiefungen kraterförmig sich in Richtung auf die Trägerschicht (5) verjüngend ausgebildet sind.

23. Prophylaxeartikel (1) nach Anspruch 22, **dadurch gekennzeichnet, dass** Wände der kraterförmigen Vertiefungen einen Neigungswinkel, bezogen auf die Normale auf die Gleitschicht (4) aufweisen ausgewählt aus einem Bereich mit einer unteren Grenze von 30 °, insbesondere 42 °, vorzugsweise 47 °, und einer oberen Grenze von 80 °, insbesondere 75 °, vorzugsweise 60 °.

24. Prophylaxeartikel (1) nach einem der Ansprüche 1 bis 23, **dadurch gekennzeichnet, dass** eine Menge des Wirkstoffes und/oder Farbstoffes so bemessen ist, dass der Wirkstoff und/oder Farbstoff über die gesamte Tragedauer des Prophylaxeartikels in vorzugsweise zumindest annähernd gleichmäßigen Dosen abgegeben wird.

25. Prophylaxeartikel (1) nach einem der Ansprüche 1 bis 24, **dadurch gekennzeichnet, dass** der Wirkstoff und/oder Farbstoff in Wasser eine Löslichkeit bei 20 °C ausgewählt aus einem Bereich mit einer unteren Grenze von 1 g/l, vorzugsweise 3 g/l, insbesondere 4,5 g/l, und einer oberen Grenze von 20 g/l, vorzugsweise 15 g/l, insbesondere 8 g/l, aufweist.

26. Prophylaxeartikel (1) nach einem der Ansprüche 1 bis 25, **dadurch gekennzeichnet, dass** eine Lösung des Wirkstoffes und/oder Farbstoffes im Partikel (11) einen pH-Wert, ausgewählt aus einem Bereich von 5,5 bis 7,5, aufweist.

27. Prophylaxeartikel (1) nach einem der Ansprüche 1 bis 26, **dadurch gekennzeichnet, dass** die Erhebungen zumindest großteils netzförmig mit miteinander verbundenen Stegen ausgebildet sind.

28. Prophylaxeartikel (1) nach Anspruch 27, **dadurch gekennzeichnet, dass** eine Höhe zumindest eines Teils der Stege einen Wert aufweist, der im Bereich zwischen 25 % und 100 %, vorzugsweise 33 % und 75 %, insbesondere 40 % und 60 %, der Gesamtdicke der Gleitschicht (4) beträgt.

29. Verfahren zur Herstellung eines mehrschichtigen Prophylaxeartikels (1), insbesondere eines medizinischen Handschuhs (2), bei dem aus einem elastomeren Material, vorzugsweise aus Synthese- oder Naturlatex, zumindest eine Trägerschicht (5) hergestellt wird, die eine innere Oberfläche (7) und eine äußere Oberfläche (6) aufweist und zumindest ein Wirkstoff in Partikeln (11) aufgebracht wird, **dadurch gekennzeichnet, dass** auf die innere Oberfläche (7) der Trägerschicht (5) eine Gleitschicht (4) aus einem polymeren Material mit einer inneren Oberfläche (9) und einer, der inneren Oberfläche (7) der Trägerschicht (5) zugewandten, äußeren Oberfläche (8) aufgebracht wird, wobei auf der inneren Oberfläche (7) der Trägerschicht (5) und/oder zwischen der Trägerschicht (5) und der Gleitschicht (4) und/oder in der Gleitschicht (4) und/oder auf der äußeren Oberfläche (8) der Gleitschicht (4) zumindest ein Wirkstoff und/oder Farbstoff in Partikeln (11) aufgebracht wird, insbesondere durch Tauchen oder Aufsprühen, wobei Partikel (11) mit einem maximalen Durchmesser aus einem Bereich mit einer oberen Grenzen von 500 µm, insbesondere 400 µm, vorzugsweise 300 µm und einer unteren Grenze von 10 µm, vorzugsweise 30 µm, insbesondere 40 µm, ausgewählt werden und in der Gleitschicht regelmäßig wiederkehrende Erhöhungen bzw. Vertiefungen mit unregelmäßiger Form durch raschen Flüssigkeitsentzug aus der Gleitschicht (4) hergestellt werden, wobei sich ein Anteil der Vertiefungen ausgewählt aus einem Bereich mit einer unteren Grenze von 20 %, insbesondere 35 %, vorzugsweise 40 %, und einer oberen Grenze von 95 %, insbesondere 80 %, vorzugsweise 75 %, bezogen auf die Gesamtzahl der Vertiefungen, über die gesamte Dicke der Gleitschicht (4) erstreckt.

30. Verfahren nach Anspruch 29, **dadurch gekennzeichnet, dass** Partikel (11) mit einem Durchmesser, ausgewählt aus einem Bereich mit einer oberen Grenze von 250 µm, vorzugsweise 200 µm, insbesondere 150 µm, und einer unteren Grenze von 50 µm, vorzugsweise 80 µm, insbesondere 100 µm, aufgebracht werden.

31. Verfahren nach Anspruch 29 oder 30, **dadurch gekennzeichnet, dass** die Partikel (11) und/oder die Schicht in Form eines heterogenen Gemisches, insbesondere einer Suspension bzw. Dispersion, aufgebracht werden bzw. wird.

32. Verfahren nach Anspruch 31, **dadurch gekennzeichnet, dass** durch das heterogene Gemisch in dem zumindest einen Teilbereich die Gleitschicht (4) ausgebildet wird.

33. Verfahren nach einem der Ansprüche 29 bis 32, **dadurch gekennzeichnet, dass** eine Konzentration an Partikel (11) im heterogenen Gemisch aus einem Bereich mit einer unteren Grenze von 1 %, insbesondere 2 %, vorzugsweise 5 %, und einer oberen Grenze von 50 %, vorzugsweise 40 %, insbesondere 30 %, verwendet wird.

34. Verfahren nach Anspruch 22, **dadurch gekennzeichnet, dass** die Konzentration der Partikel (11) im heterogenen Gemisch aus einem Bereich mit einer unteren Grenze von 6 %, vorzugsweise 7 %, insbesondere 10 % und einer oberen Grenze von 25 %, vorzugsweise 20 %, insbesondere 15 %, ausgewählt wird.

35. Verfahren nach einem der Ansprüche 29 bis 34, **dadurch gekennzeichnet, dass** der Flüssigkeitsentzug innerhalb einer Zeitspanne mit einer unteren Grenze von 10 s, insbesondere 25 s, vorzugsweise 50 s, und einer oberen Grenze von 20 min, insbesondere 15 min, vorzugsweise 10 min, erfolgt.

36. Verfahren nach einem der Ansprüche 29 bis 34, **dadurch gekennzeichnet, dass** der Flüssigkeitsentzug bei einer Temperatur, ausgewählt aus einem Bereich mit einer unteren Grenze von 60 °C, insbesondere 66 °C, vorzugsweise 70 °C, und einer oberen Grenze von 150 °C, insbesondere 125 °C, vorzugsweise 110 °C, durchgeführt wird.

37. Verfahren nach einem der Ansprüche 29 bis 36, **dadurch gekennzeichnet, dass** Partikel (11) mit einer wasserlöslichen Hülle verwendet werden.

38. Verfahren nach einem der Ansprüche 29 bis 36, **dadurch gekennzeichnet, dass** Partikel (11) mit einer wasserunlöslichen Hülle verwendet werden.

39. Verfahren nach einem der Ansprüche 29 bis 38, **dadurch gekennzeichnet, dass** als Wirkstoff ein Wirkstoff mit antibakterieller bzw. antiviraler bzw. antimykotischer bzw. antitranspiranter bzw. spermizider bzw. pflegender Wirkung verwendet wird.

40. Verfahren nach einem der Ansprüche 29 bis 39, **dadurch gekennzeichnet, dass** der Wirkstoff ausgewählt wird aus einer Gruppe umfassend Chlorhexidin, z.B. ein Gluconat, ein Acetat, ein Hydrochlorid, Nonoxinol 9 und Aloe Vera.

41. Verfahren nach einem der Ansprüche 29 bis 40, **dadurch gekennzeichnet, dass** der Wirkstoff aus einer Gruppe umfassend Vitamine, Pflanzeninhaltsstoffe, insbesondere sekundäre Pflanzeninhaltsstoffe, ausgewählt wird.

42. Verfahren nach Anspruch 41, **dadurch gekennzeichnet, dass** Vitamin aus einer Gruppe umfassend Verbindungen mit Retinoidstruktur (Vitamine A), Vitamin-B-Komplex, Ascorbinsäure (Vitamine C), Calciferole (Vitamine D), Tocopherole (Vitamine E), Vitamine K, Flavanoide und Biotin ausgewählt wird.

43. Verfahren nach einem der Ansprüche 29 bis 42, **dadurch gekennzeichnet, dass** der zumindest eine Wirkstoffe und/oder Farbstoffe in einer Konzentration im Partikel (11) verwendet wird, die aus einem Bereich mit einer unteren Grenze von 1%, vorzugsweise 2 %, insbesondere 5 %, und einer oberen Grenze von 20 %, vorzugsweise 15 %, insbesondere 10 %, ausgewählt ist.

44. Verfahren nach einem der Ansprüche 29 bis 43, **dadurch gekennzeichnet, dass** durch das Auftragen der Partikel (11) in dem zumindest einen Teilbereich die Gleitschicht (4) gebildet wird.

45. Verfahren nach einem der Ansprüche 29 bis 44, **dadurch gekennzeichnet, dass** das Material für die Gleitschicht (4) solange aufgetragen wird, bis diese eine Dicke, ausgewählt aus einem Bereich mit einer unteren Grenze von 30 µm, vorzugsweise 40 µm, insbesondere 50 µm, und einer oberen Grenze von 500 µm, vorzugsweise 400 µm, insbesondere 300 µm, aufweist.

46. Verfahren nach Anspruch 45, **dadurch gekennzeichnet, dass** die Dicke der Gleitschicht (4) ausgewählt wird aus einem Bereich mit einer unteren Grenze von 55 µm, vorzugsweise 60 µm, insbesondere 75 µm, und einer oberen Grenze von 200 µm, vorzugsweise 150 µm, insbesondere 110 µm.

47. Verfahren nach einem der Ansprüche 29 bis 46, **dadurch gekennzeichnet, dass** die Zeit während der der Flüssigkeitsentzug stattfindet so bemessen ist, dass Vertiefungen mit einen maximalen Durchmesser, in Draufsicht gesehen, ausgewählt aus einem Bereich mit einer oberen Grenze von 30 µm, vorzugsweise 25 µm, insbesondere 20 µm, und einer unteren Grenze von 1 µm, vorzugsweise 5 µm, insbesondere 10 µm, entstehen.

48. Verfahren nach einem der Ansprüche 29 bis 47, **dadurch gekennzeichnet, dass** die Zeit während der der Flüssigkeitsentzug stattfindet so bemessen ist, dass kraterförmige, sich in Richtung auf die Trägerschicht (5) verjüngende Vertiefungen ausgebildet werden.

49. Verfahren nach einem der Ansprüche 29 bis 48, **dadurch gekennzeichnet, dass** ein Wirkstoff und/oder Farbstoff verwendet wird, der in Wasser eine Löslichkeit bei 20 °C ausgewählt aus einem Bereich mit einer unteren Grenze von 1 g/l, vorzugsweise 3 g/l, insbesondere 4,5 g/l, und einer oberen Grenze von 20 g/l, vorzugsweise 15 g/l, insbesondere 8 g/l, aufweist.

50. Verfahren nach einem der Ansprüche 29 bis 49, **dadurch gekennzeichnet, dass** eine Lösung des Wirkstoffes und/oder Farbstoffes im Partikel (11) derart eingestellt und/oder mit einem Puffer versetzt wird, dass diese einen pH-Wert, ausgewählt aus einem Bereich von 5,5 bis 7,5, aufweist bzw. beibehält.

51. Verfahren nach einem der Ansprüche 29 bis 50, **dadurch gekennzeichnet, dass** die Erhebungen zumindest großteils netzförmig mit miteinander verbundenen Stegen ausgebildet werden.

52. Verfahren nach Anspruch 51, **dadurch gekennzeichnet, dass** zumindest ein Teil der Stege mit einer Höhe, die einen Wert aufweist der im Bereich zwischen 25 % und 100 % vorzugsweise 33 % und 75 %, insbesondere 40 % und 60 %, der Gesamtdicke der Gleitschicht (4) beträgt, gebildet werden.

## Claims

1. A multi-layered device for prophylaxis (1), in particular a medical glove (2), produced from an elastomeric base layer (5), such as a synthetic or natural latex, for example, with an internal and an external surface (6, 7), with at least one active substance within particles (11), in particular microcapsules, **characterized in that** at least one section of the internal surface (7) is provided with an anti-friction layer (4) produced from a polymeric material with an internal surface (9) and an external surface (8) facing the internal surface (7) of the base layer (5), wherein particles (11) with at least one active substance and/or dye with a maximum diameter selected from a range with an upper limit of 500 µm, in particular 400 µm, preferably 300 µm, and a lower limit of 10 µm, preferably 30 µm, in particular 40 µm, are disposed in at least one section on the internal surface (9) of the base layer (5) and/or between the base layer (5) and the anti-friction layer (4) and/or in the anti-friction layer (4) and/or on the internal surface (9) of the anti-friction layer (4), and the anti-friction layer (4) is provided with regularly recurring raised areas or depressed areas with an irregular shape, produced by rapidly removing liquid from the anti-friction layer (4), wherein a proportion of the depressed areas selected from a range with a lower limit of 20 %, in particular 35 %, preferably 40 %, and an upper limit of 95 %, in particular 80 %, preferably 75 %, with respect to the total number of depressed areas, extends through the entire thickness of the anti-friction layer (4).

2. The device for prophylaxis (1) as claimed in claim 1, **characterized in that** the diameter of the particles (11) is selected from a range with an upper limit of 250 µm, preferably 200 µm, in particular 150 µm, and a lower limit of 50 µm, preferably 80 µm in particular 100 µm.

3. The device for prophylaxis (1) as claimed in claim 1 or 2, **characterized in that** the diameter of the particles (11) is at least 80 %, preferably at least 85 %, in particular at least 90 % of the thickness of the anti-friction layer (4).

4. The device for prophylaxis (1) as claimed in claim 3, **characterized in that** the diameter of the particle (11) is the same size as the thickness of the anti-friction layer (4).

5. The device for prophylaxis (1) as claimed in claim 3, **characterized in that** the diameter of the particle (11) is larger than the thickness of the anti-friction layer (4).

6. The device for prophylaxis (1) as claimed in one of claims 1 to 5, **characterized in that** the section encompasses the region of the distal forearm and/or the carpal bones and/or the metacarpals and/or the base, middle and terminal phalanges of the fingers.

7. The device for prophylaxis (1) as claimed in one of claims 1 to 6, **characterized in that** the particles (11) and/or the layer are applied to both the palm side and dorsal side in at least one section.

8. The device for prophylaxis (1) as claimed in one of claims 1 to 7, **characterized in that** the section extends over a region of the internal surface (7) of the base layer (5) and/or between the base layer (5) and the anti-friction layer (4) and/or in the anti-friction layer (4) and/or on the internal surface (9) of the anti-friction layer (4) to an extent in a range from a lower limit of 40 %, preferably 50 %, in particular 60 %, and an upper limit of 100 %, preferably 80 %, in particular 70 %.

9. The device for prophylaxis (1) as claimed in one of claims 1 to 8, **characterized in that** the particles (11) and/or the layer are a different colour from the base layer (5) or anti-friction layer (4).

10. The device for prophylaxis (1) as claimed in one of claims 1 to 9, **characterized in that** the particles (11) are water-insoluble.

11. The device for prophylaxis (1) as claimed in one of claims 1 to 9, **characterized in that** the particles (11) are water-soluble.

12. The device for prophylaxis (1) as claimed in one of claims 1 to 11, **characterized in that** the active substance has an antibacterial, antiviral, antifungal, antiperspirant, spermicidal or protective action, respectively.

13. The device for prophylaxis (1) as claimed in claim 12, wherein the active substance is selected from a group comprising of chlorhexidine, e.g. gluconate, acetate, hydrochloride, nonoxinol 9 and aloe vera.

14. The device for prophylaxis (1) as claimed in one of claims 1 to 13, **characterized in that** the active substance is selected from a group comprising of vitamins and plant extracts, in particular secondary plant extracts.

15. The device for prophylaxis (1) as claimed in claim 14, **characterized in that** vitamins are selected from a group comprising compounds with a retinoid structure (vitamin A), vitamin B-complex, ascorbic acid (vitamin C), calciferols (vitamin D), tocopherols (vitamin E), vitamin K, flavonoids and biotin.

16. The device for prophylaxis (1) as claimed in one of claims 12 to 15, **characterized in that** the concentration of the at least one active substance and/or dye in the particle (11) is selected from a range with a lower limit of 1 %, preferably 2 %, in particular 5 %, and an upper limit of 20 %, preferably 15 %, in particular 10 %.

17. The device for prophylaxis (1) as claimed in one of claims 1 to 16, **characterized in that** a shell of the particles (11) is pressure-sensitive.

18. The device for prophylaxis (1) as claimed in one of claims 1 to 17, **characterized in that** the particles (11) form the anti-friction layer (4) in the at least one section.

19. The device for prophylaxis (1) as claimed in one of claims 1 to 18, **characterized in that** a thickness of the anti-friction layer (4) is selected from a range with a lower limit of 30 µm, preferably 40 µm, in particular 50 µm, and an upper limit of 500 µm, preferably 400 µm, in particular 300 µm.

20. The device for prophylaxis (1) as claimed in claim 19, **characterized in that** the thickness of the anti-friction layer (4) is selected from a range with a lower limit of 55 µm, preferably 60 µm, in particular 75 µm, and an upper limit of 200 µm, preferably 150 µm, in particular 110 µm.

21. The device for prophylaxis (1) as claimed in one of claims 1 to 20, **characterized in that** the depressed areas have a maximum diameter, as seen in plan view, selected from a range with an upper limit of 30 µm, preferably 25 µm, in particular 20 µm, and a lower limit of 1 µm, preferably 5 µm, in particular 10 µm.

22. The device for prophylaxis (1) as claimed in one of claims 1 to 21, **characterized in that** the depressed areas are crater-shaped and taper in the direction towards the base layer (5).

23. The device for prophylaxis (1) as claimed in claim 22, wherein walls of the crater-shaped depressed areas subtend an angle with the line perpendicular to the anti-friction layer (4) selected from a range with a lower limit of 30°, in particular 42°, preferably 47°, and an upper limit of 80°, in particular 75°, preferably 60°.

24. The device for prophylaxis (1) as claimed in one of claims 1 to 23, **characterized in that** a quantity of the active substance and/or dye is selected such that the active substance and/or dye is preferably released in at least substantially uniform doses throughout the entire time period during which the device for prophylaxis (1) is being worn.

25. The device for prophylaxis (1) as claimed in one of claims 1 to 24, **characterized in that** the active substance and/or dye has a solubility in water at 20 °C which is selected from a range with a lower limit of 1 g/L, preferably 3 g/L, in particular 4.5 g/L, and an upper limit of 20 g/L, preferably 15 g/L, in particular 8 g/L.

26. The device for prophylaxis (1) as claimed in one of claims 1 to 25, **characterized in that** a solution of the active substance and/or dye in the particle (11) has a pH selected from a range of 5.5 to 7.5.

27. The device for prophylaxis (1) as claimed in one of claims 1 to 26, **characterized in that** the raised areas are arranged in an at least predominantly network-like arrangement with interconnecting webs.

28. The device for prophylaxis (1) as claimed in claim 27, **characterized in that** a height of at least a portion of the webs has a value in the range between 25 % and 100 %, preferably 33 % and 75 %, in particular 40 % and 60 %, of the total thickness of the anti-friction layer (4).

29. A process for the production of a multi-layered device for prophylaxis (1), in particular a medical glove (2), in which at least a base layer (5) is produced from an elastomeric material, preferably synthetic or natural rubber, which has an internal surface (7) and an external surface (6) upon which at least one active substance within particles (11) is applied, **characterized in that** an anti-friction layer (4) produced from a polymeric material is applied to the internal surface (7) of the base layer (5) has an internal surface (9) and an external surface (8) facing the internal surface (7) of the base layer (5), wherein at least one active substance and/or dye in particles (11) is applied to the internal surface (7) of the base layer (5) and/or between the base layer (5) and the anti-friction layer (4) and/or in the anti-friction layer (4) and/or on the external surface (8) of the anti-friction layer (4), in particular by dipping or spraying, wherein particles (11) with a maximum diameter selected from a range with an upper limit of 500 µm, in particular 400 µm, preferably 300µm, and a lower limit of 10 µm, preferably 30 µm, in particular 40 µm are selected and regularly recurring raised areas or depressed areas with an irregular shape are produced in an anti-friction layer by rapidly removing liquid from the anti-friction layer (4), wherein a proportion of the depressed areas selected from a range with a lower limit of 20 %, in particular 35 %, preferably 40 %, and an upper limit of 95 %, in particular 80 %, preferably 75 %, with respect to the total number of depressed areas, extends through the entire thickness of the anti-friction layer (4).

30. The process as claimed in claim 29, **characterized in that** that particles (11) are applied which have a diameter selected from a range with an upper limit of 250 µm, preferably 200 µm, in particular 150 µm, and a lower limit of 50 µm, preferably 80 µm, in particular 100 µm.

31. The process as claimed in claim 29 or 30, **characterized in that** the particles (11) and/or the layer is or are applied in the form of a heterogeneous mixture, in particular a suspension or dispersion.

32. The process as claimed in claim 31, **characterized in that** at least one section of the anti-friction layer (4) is formed by the heterogeneous mixture.

33. The process as claimed in one of claims 29 to 32, **characterized in that** a concentration of particles (11) in the heterogeneous mixture is used which is selected from a range with a lower limit of 1 %, in particular 2 %, preferably 5 %, and an upper limit of 50 %, preferably 40 %, in particular 30 %.

34. The process as claimed in claim 22, **characterized in that** the concentration of the particles (11) in the heterogeneous mixture is selected from a range with a lower limit of 6 %, preferably 7 %, in particular 10 %, and an upper limit of 25 %, preferably 20 %, in particular 15 %.

35. The process as claimed in one of claims 29 to 34, **characterized in that** the liquid is removed within a time period with a lower limit of 10 s, in particular 25 s, preferably 50 s, and an upper limit of 20 min, in particular 15 min, preferably 10 min.

36. The process as claimed in one of claims 29 to 34, **characterized in that** the liquid is removed at a temperature selected from a range with a lower limit of 60 °C, in particular 66 °C, preferably 70 °C, and an upper limit of 150 °C, in particular 125 °C, preferably 110 °C.

37. The process as claimed in one of claims 29 to 36, **characterized in that** particles (11) with a water-soluble shell are used.

38. The process as claimed in one of claims 29 to 36, **characterized in that** particles (11) with a water-insoluble shell are used.

39. The process as claimed in one of claims 29 to 38, **characterized in that** the active substance is a substance with an antibacterial, antiviral, antifungal, antiperspirant, spermicidal or protective action, respectively.

40. The process as claimed in one of claims 29 to 39, **characterized in that** the active substance is selected from a group comprising of chlorhexidine, e.g. gluconate, acetate, hydrochloride, nonoxinol 9 and aloe vera.

41. The process as claimed in one of claims 29 to 40, **characterized in that** the substance is selected from a group comprising vitamins and plant extracts, in particular secondary plant extracts.

42. The process as claimed in claim 41, **characterized in that** the vitamins are selected from a group comprising compounds with a retinoid structure (vitamin A), vitamin B-complex, ascorbic acid (vitamin C), calciferols (vitamin D), tocopherols (vitamin E), vitamin K, flavonoids and biotin.

43. The process as claimed in one of claims 29 to 42, **characterized in that** the at least one active substance and/or dye is contained in the particle (11) in a concentration selected from a range with a lower limit of 1 %, preferably 2 %, in particular 5 %, and an upper limit of 20 %, preferably 15 %, in particular 10 %.

44. The process as claimed in one of claims 29 to 43, **characterized in that** the anti-friction layer (4) is formed by applying the particles (11) to the at least one section.

45. The process as claimed in one of claims 29 to 44, **characterized in that** the material used for the anti-friction layer (4) is applied until the latter has a thickness selected from a range with a lower limit of 30 µm, preferably 40 µm, in particular 50 µm, and an upper limit of 500 µm, preferably 400 µm, in particular 300 µm.

46. The process as claimed in claim 45, **characterized in that** the thickness of the anti-friction layer (4) is selected from a range with a lower limit of 55 µm, preferably 60 µm, in particular 75 µm, and an upper limit of 200 µm, preferably 150 µm, in particular 110 µm.

47. The process as claimed in one of claims 29 to 46, **characterized in that** the time during which liquid is removed is selected so that the depressed areas are produced with a maximum diameter, as seen in plan view, selected from a range with an upper limit of 30 µm, preferably 25 µm, in particular 20 µm, and a lower limit of 1 µm, preferably 5 µm, in particular 10 µm.

48. The process as claimed in one of claims 29 to 47, **characterized in that** the time period during which the liquid is removed is selected so that crater-shaped depressed areas are formed which taper in the direction towards the base layer (5).

49. The process as claimed in one of claims 29 to 48, **characterized in that** an active substance and/or a dye is used which has a solubility in water at 20 °C that is selected from a range with a lower limit of 1 g/L, preferably 3 g/L, in particular 4.5 g/L, and an upper limit of 20 g/L, preferably 15 g/L, in particular 8 g/L.

50. The process as claimed in one of claims 29 to 49, **characterized in that** a solution of the active substance and/or dye in the particle (11) is adjusted and/or a buffer is added in a manner such that it has or maintains a pH selected from a range of 5.5 to 7.5.

51. The process as claimed in one of claims 29 to 50, **characterized in that** the raised areas form an at least substantially network-like pattern with interconnecting webs.

52. The process as claimed in claim 51, **characterized in that** at least a portion of the webs are formed with a height which has a value in the range between 25 % and 100 %, preferably 33 % and 75 %, in particular 40 % and 60 %, of the total thickness of the anti-friction layer (4).

## Revendications

1. Article prophylactique multicouche (1), en particulier gant (2) à usage médical, constitué d'une couche support élastomère (5), telle que par exemple un latex synthétique ou naturel, ayant une surface intérieure et une surface extérieure (6, 7), comprenant au moins une matière active à l'intérieur de particules (11), en particulier des microcapsules, **caractérisé en ce que** sur la surface intérieure (7), au moins dans une zone partielle, est disposée une couche de glissement (4) en un matériau polymère, ayant une surface intérieure (9) et une surface extérieure (8) dirigée vers la surface intérieure (7) de la couche support (5) ; sur la surface intérieure (9) de la couche support (5) et/ou entre la couche support (5) et la couche de glissement (4) et/ou dans la couche de glissement (4) et/ou sur la surface intérieure (9) de la couche de glissement (4), au moins dans la zone partielle, sont disposées des particules (11) comprenant au moins une matière active et/ou un colorant, ayant un diamètre maximal choisi dans une plage ayant une limite supérieure de 500 µm, en particulier de 400 µm, de préférence de 300 µm, et une limite inférieure de 10 µm, de préférence de 30 µm, en particulier de 40 µm ; et la couche de glissement (4) présente des surélévations ou des renfoncements régulièrement répétitifs, ayant une forme irrégulière, fabriqués par extraction rapide de liquide à partir de la couche de glissement (4), une partie des renfoncements, choisie dans une plage ayant une limite inférieure de 20 %, en particulier de 35 %, de préférence de 40 %, et une limite supérieure de 95 %, en particulier de 80 %, de préférence de 75 % en poids, par rapport au nombre total des renfoncements, s'étendant sur la totalité de l'épaisseur de la couche de glissement (4).

2. Article prophylactique (1) selon la revendication 1, **caractérisé en ce que** le diamètre des particules (11) est choisi dans une plage ayant une limite supérieure de 250 µm, de préférence de 200 µm, en particulier de 150 µm, et une limite inférieure de 50 µm, de préférence de 80 µm, en particulier de 100 µm.

3. Article prophylactique (1) selon la revendication 1 ou 2, **caractérisé en ce que** le diamètre des particules (11) est d'au moins 80 %, de préférence d'au moins 85 %, en particulier d'au moins 90 % de l'épaisseur de la couche de glissement (4).

4. Article prophylactique (1) selon la revendication 3, **caractérisé en ce que** le diamètre des particules (11) est égal à l'épaisseur de la couche de glissement (4).

5. Article prophylactique (1) selon la revendication 3, **caractérisé en ce que** le diamètre de particule (11) est supérieur à l'épaisseur de la couche de glissement (4).

6. Article prophylactique (1) selon l'une des revendications 1 à 5, **caractérisé en ce que** la zone partielle comprend la zone de l'avant-bras distal et/ou du carpe et/ou des os métacarpiens et/ou des phalanges proximales, médianes et distales des doigts.

7. Article prophylactique (1) selon l'une des revendications 1 à 6, **caractérisé en ce que** les particules (11) et/ou la couche sont appliquées, dans au moins une zone partielle, tant d'une manière palmaire que d'une manière dorsale.

8. Article prophylactique (1) selon l'une des revendications 1 à 7, **caractérisé en ce que** la zone partielle s'étend sur une zone de la surface intérieure (7) de la couche supérieure (5) et/ou entre la couche support (5) et la couche de glissement (4) et/ou dans la couche de glissement (4) et/ou sur la surface intérieure (9) de la couche de glissement (4), ayant une limite inférieure de 40 %, de préférence de 50 %, en particulier de 60 %, et une limite supérieure de 100 %, de préférence de 80 %, en particulier de 70 %.

9. Article prophylactique (1) selon l'une des revendications 1 à 8, **caractérisé en ce que** les particules (11) et/ou la couche présentent une couleur différente de celle de la couche support (5) ou de la couche de glissement (4).

10. Article prophylactique (1) selon l'une des revendications 1 à 9, **caractérisé en ce que** les particules (11) sont insolubles dans l'eau.

11. Article prophylactique (1) selon l'une des revendications 1 à 9, **caractérisé en ce que** les particules (11) sont solubles dans l'eau.

12. Article prophylactique (1) selon l'une des revendications 1 à 11, **caractérisé en ce que** la matière active présente un effet antibactérien ou antiviral ou antimycotique ou antitranspirant ou spermicide, ou curatif.

13. Article prophylactique (1) selon la revendication 12, **caractérisé en ce que** la matière active est choisie dans un groupe comprenant la chlorhexidine, p.ex. un gluconate, un acétate, un chlorhydrate, le Nonoxinol 9 et Aloe vera.

14. Article prophylactique (1) selon l'une des revendications 1 à 13, **caractérisé en ce que** la matière active est choisie dans un groupe comprenant les vitamines, les constituants végétaux, en particulier les constituants végétaux secondaires.

15. Article prophylactique (1) selon la revendication 14, **caractérisé en ce que** les vitamines sont choisies dans un groupe comprenant les composés ayant une structure de rétinoïde (vitamines A), le complexe de vitamines B, l'acide ascorbique (vitamines C), les calciférols (vitamines D), les tocophérols (vitamines E), les vitamines K, les flavonoïdes et la biotine.

16. Article prophylactique (1) selon l'une des revendications 12 à 15, **caractérisé en ce que** la concentration de l'au moins une matière active et/ou de l'au moins un colorant dans la particule (11) est choisie dans une plage ayant une limite inférieure de 1 %, de préférence de 2 %, en particulier de 5 %, et une limite supérieure de 20 %, de préférence de 15 %, en particulier de 10 %.

17. Article prophylactique (1) selon l'une des revendications 1 à 16, **caractérisé en ce qu'**une enveloppe des particules (11) est sensible à la pression.

18. Article prophylactique (1) selon l'une des revendications 1 à 17, **caractérisé en ce que** les particules (11) forment la couche de glissement (4) dans l'au moins une zone partielle.

19. Article prophylactique (1) selon l'une des revendications 1 à 18, **caractérisé en ce qu'**une épaisseur de la couche de glissement (4) est choisie dans une plage ayant une limite inférieure de 30 µm, de préférence de 40 µm, en particulier de 50 µm, et une limite supérieure de 500 µm, de préférence de 400 µm, en particulier de 300 µm.

20. Article prophylactique (1) selon la revendication 19, **caractérisé en ce que** l'épaisseur de la couche de glissement (4) est choisie dans une plage ayant une limite inférieure de 55 µm, de préférence de 60 µm, en particulier de 75 µm, et une limite supérieure de 200 µm, de préférence de 150 µm, en particulier de 110 µm.

21. Article prophylactique (1) selon l'une des revendications 1 à 20, **caractérisé en ce que** les renfoncements présentent, en vue de dessus, un diamètre maximal choisi dans une plage ayant une limite supérieure de 30 µm, de préférence de 25 µm, en particulier de 20 µm, et une limite inférieure de 1 µm, de préférence de 5 µm, en particulier de 10 µm.

22. Article prophylactique (1) selon l'une des revendications 1 à 21, **caractérisé en ce que** les renfoncements sont conçus en se rétrécissant en forme de cratère dans la direction allant vers la couche support (5).

23. Article prophylactique (1) selon la revendication 22, **caractérisé en ce que** les parois des renfoncements en forme de cratère présentent un angle d'inclinaison, par rapport à la normale à la couche de glissement (4), choisi dans une plage ayant une limite inférieure de 30°, en particulier de 42°, de préférence de 47°, et une limite supérieure de 80°, en particulier de 75°, de préférence de 60°.

24. Article prophylactique (1) selon l'une des revendications 1 à 23, **caractérisé en ce qu'**une quantité de la matière active et/ou du colorant est mesurée de telle sorte que la matière active et/ou le colorant soient libérés sur la totalité de la durée de port de l'article prophylactique, à des doses de préférence au moins approximativement uniformes.

25. Article prophylactique (1) selon l'une des revendications 1 à 24, **caractérisé en ce que** la matière active et/ou le colorant présentent dans l'eau une solubilité à 20°C choisie dans une plage ayant une limite inférieure de 1 g/l, de préférence de 3 g/l, en particulier de 4,5 g/l, et une limite supérieure de 20 g/l, de préférence de 15 g/l, en particulier de 8 g/l.

26. Article prophylactique (1) selon l'une des revendications 1 à 25, **caractérisé en ce qu'**une solution de la matière active et/ou du colorant dans la particule (11) présente un pH choisi dans une plage de 5,5 à 7,5.

27. Article prophylactique (1) selon l'une des revendications 1 à 26, **caractérisé en ce que** les surélévations sont, au moins en majorité, configurées en forme de réseau, avec des nervures reliées les unes aux autres.

28. Article prophylactique (1) selon la revendication 27, **caractérisé en ce qu'**une hauteur d'au moins une partie des nervures présentent une valeur qui est comprise dans la plage entre 25 % et 100 %, de préférence entre 33 % et 75 %, en particulier entre 40 % et 60 % de l'épaisseur totale de la couche de glissement (4).

29. Procédé de fabrication d'un article prophylactique multicouche (1), en particulier d'un gant à usage médical (2), dans lequel, à partir d'un matériau élastomère, de préférence d'un latex synthétique ou naturel, on fabrique au moins une couche support (5), qui présente une surface intérieure (7) et une surface extérieure (6), et au moins une matière active est appliquée dans des particules (11), **caractérisé en ce que**, sur la surface intérieure (7) de la couche support (5), on applique une couche de glissement (4) en un matériau polymère, ayant une surface intérieure (9) et une surface extérieure (8) dirigée vers la surface intérieure (7) de la couche support (5) ; sur la surface intérieure (7) de la couche support (5) et/ou entre la couche support (5) et la couche de glissement (4) et/ou dans la couche de glissement (4) et/ou sur la couche extérieure (8) de la couche de glissement (4), on applique au moins une matière active et/ou un colorant dans les particules (11), en particulier par immersion ou pulvérisation, des particules (11) ayant un diamètre maximal compris dans une plage ayant une limite supérieure de 500 µm, en particulier de 400 µm, de préférence de 300 µm, et une limité inférieure de 10 µm, de préférence de 30 µm, en particulier de 40 µm, étant choisies, et des surélévations ou des renfoncements, régulièrement répétitifs dans la couche de glissement, ayant une forme irrégulière, étant fabriqués par extraction rapide d'un liquide à partir de la couche de glissement (4) ; une proportion des renfoncements étant choisie dans une plage ayant une limite inférieure de 20 %, en particulier de 35 %, de préférence de 40 %, et une limite supérieure de 95 %, en particulier de 80 %, de préférence de 75 %, par rapport au nombre total des renfoncements, s'étendant sur la totalité de l'épaisseur de la couche de glissement (4).

30. Procédé selon la revendication 29, **caractérisé en ce qu'**on applique des particules (11) ayant un diamètre choisi dans une plage ayant une limite supérieure de 250 µm, de préférence de 200 µm, en particulier de 150 µm, et une limite inférieure de 50 µm, de préférence de 80 µm, en particulier de 100 µm.

31. Procédé selon la revendication 29 ou 30, **caractérisé en ce qu'**on applique les particules (11) et/ou la couche sous forme d'un mélange hétérogène, en particulier d'une suspension ou d'une dispersion.

32. Procédé selon la revendication 31, **caractérisé en ce que** la couche de glissement (4) est obtenue par le mélange hétérogène dans l'au moins une zone partielle.

33. Procédé selon l'une des revendications 29 à 32, **caractérisé en ce qu'**on utilise une concentration de particules (11) dans le mélange hétérogène, comprise dans une plage ayant une limite inférieure de 1 %, en particulier de 2 %, de préférence de 5 %, et une limite supérieure de 50 %, de préférence de 40 %, en particulier de 30 %.

34. Procédé selon la revendication 22, **caractérisé en ce que** la concentration des particules (11) dans le mélange hétérogène est choisie dans une plage ayant une limite inférieure de 6 %, de préférence de 7 %, en particulier de 10 %, et une limite supérieure de 25 %, de préférence de 20 %, en particulier de 15 %.

35. Procédé selon l'une des revendications 29 à 34, **caractérisé en ce que** l'extraction d'un liquide a lieu sur un laps de temps ayant une limite inférieure de 10 s, en particulier de 25 s, de préférence de 50 s, et une limite supérieure de 20 min, en particulier de 15 min, de préférence de 10 min.

36. Procédé selon l'une des revendications 29 à 34, **caractérisé en ce que** l'extraction du liquide est mise en oeuvre à une température choisie dans une plage ayant une limite inférieure de 60°C, en particulier de 66°C, de préférence de 70°C, et une limite supérieure de 150°C, en particulier de 125°C, de préférence de 110°C.

37. Procédé selon l'une des revendications 29 à 36, **caractérisé en ce qu'**on utilise des particules (11) ayant une enveloppe soluble dans l'eau.

38. Procédé selon l'une des revendications 29 à 36, **caractérisé en ce qu'**on utilise des particules (11) ayant une enveloppe insoluble dans l'eau.

39. Procédé selon l'une des revendications 29 à 38, **caractérisé en ce qu'**on utilise en tant que matière active une matière active ayant un effet antibactérien ou antiviral ou antimycotique ou antitranspirant ou spermicide ou curatif.

40. Procédé selon l'une des revendications 29 à 39, **caractérisé en ce que** la matière active est choisie dans un groupe comprenant la chlorhexidine, p.ex. un gluconate, un acétate, un chlorhydrate, le Nonoxinol 9 et Aloe vera.

41. Procédé selon l'une des revendications 29 à 40, **caractérisé en ce que** la matière active est choisie dans un groupe comprenant les vitamines, les constituants végétaux, en particulier les constituants végétaux secondaires.

42. Procédé selon la revendication 41, **caractérisé en ce que** la vitamine est choisie dans un groupe comprenant des composés à structure de rétinoïde (vitamines A), le complexe de vitamines B, l'acide ascorbique (vitamines C), les calciférols (vitamines D), les tocophérols (vitamines E), les vitamines K, les flavonoïdes et la biotine.

43. Procédé selon l'une des revendications 29 à 42, **caractérisé en ce que** l'au moins une matière active et/ou l'au moins un colorant sont utilisés à une concentration dans la particule (11) qui est choisie dans une plage ayant une limite inférieure de 1 %, de préférence de 2 %, en particulier de 5 %, et une limite supérieure de 20 %, de préférence de 15 %, en particulier de 10 %.

44. Procédé selon l'une des revendications 29 à 43, **caractérisé en ce que** la couche de glissement (4) est formée sous l'effet de l'application des particules (11) dans l'au moins une zone partielle.

45. Procédé selon l'une des revendications 29 à 44, **caractérisé en ce que** le matériau destiné à la couche de glissement (4) est appliqué jusqu'à ce que cette dernière présente une épaisseur choisie dans une plage ayant une limite inférieure de 30 µm, de préférence de 40 µm, en particulier de 50 µm, et une limite supérieure de 500 µm, de préférence de 400 µm, en particulier de 300 µm.

46. Procédé selon la revendication 45, **caractérisé en ce que** l'épaisseur de la couche de glissement (4) est choisie dans une plage ayant une limite inférieure de 55 µm, de préférence de 60 µm, en particulier de 75 µm, et une limite supérieure de 200 µm, de préférence de 150 µm, en particulier de 110 µm.

47. Procédé selon l'une des revendications 29 à 46, **caractérisé en ce que** le temps pendant lequel a lieu l'extraction du liquide est mesuré de telle sorte qu'il se crée des renfoncements ayant un diamètre maximal, en vue de dessus, choisi dans une plage ayant une limite supérieure de 30 µm, de préférence de 25 µm, en particulier de 20 µm, et une limite inférieure de 1 µm, de préférence de 5 µm, en particulier de 10 µm.

48. Procédé selon l'une des revendications 29 à 47, **caractérisé en ce que** le temps pendant lequel a lieu l'extraction du liquide est mesuré de telle sorte qu'il se forme des renfoncements se rétrécissant en forme de cratère dans la direction allant vers la couche support (5).

49. Procédé selon l'une des revendications 29 à 48, **caractérisé en ce qu'**on utilise une matière active et/ou un colorant qui présentent dans l'eau une solubilité à 20°C choisie dans une plage ayant une limite inférieure de 1 g/l, de préférence de 3 g/l, en particulier de 4,5 g/l, et une limite supérieure de 20 g/l, de préférence de 15 g/l, en particulier de 8 g/l.

50. Procédé selon l'une des revendications 29 à 49, **caractérisé en ce qu'**on ajuste une solution de la matière active et/ou du colorant dans la particule (11), et/ou on lui ajoute un tampon, de telle sorte qu'elle présente ou conserve un pH choisi dans une plage de 5,5 à 7,5.

51. Procédé selon l'une des revendications 29 à 50, **caractérisé en ce que** les surélévations sont au moins, dans leur majorité, configurées en forme de réseau, avec des nervures reliées les unes aux autres.

52. Procédé selon la revendication 51, **caractérisé en ce qu'**au moins une partie des nervures sont formées avec une hauteur qui présente une valeur dans la plage comprise entre 25 % et 100 %, de préférence entre 33 % et 75 %, en particulier entre 40 % et 60 % de l'épaisseur totale de la couche de glissement (4).
